# EUROPEAN PATENT SPECIFICATION

(11) **EP 0 700 673 B1**
(45) Date of publication and mention of the grant of the patent: **27.03.2002**
(21) Application number: 94114177.2
(22) Date of filing: 09.09.1994
(51) Int. Cl.: A61F 13/15

(54) **Method of manufacture of an absorbent structure**
Methode zur Herstellung einer absorbierenden Struktur
Méthode de fabrication d'une structure absorbante

(43) Date of publication of application: 13.03.1996
(73) Proprietor: THE PROCTER & GAMBLE COMPANY, Cincinnati, Ohio 45202 (US)
(72) Inventor: Plischke, Manfred Dr., D-61449 Steinbach/Ts. (DE); Schmidt, Mattias Dr., D-65510 Idstein (DE)
(74) Representative: Bottema, Johan Jan

(56) References cited:
- US-A- 5 124 188
- US-A- 5 180 622

## Description

### FIELD OF THE INVENTION

The invention relates to the method of making an absorbent structure comprising a first region, a second region and a plurality of absorbent polymeric particles located in at least in one of the regions, the method comprising the step of reacting a crosslinking agent with the particles to form crosslink bonds, which crosslink bonds comprise inter-particle crosslink bonds mutually binding the particles to form an agglomerated macrostructure and/or surface crosslink bonds between molecules of the material on the surface of the particles.

### BACKGROUND OF THE INVENTION

Particulate, absorbent, polymeric compositions are capable of absorbing large quantities of liquids such as water and body exudates (e.g., urine) and are further capable of retaining such absorbed liquids under moderate pressures. The absorption characteristics of such polymeric compositions make them especially useful for incorporation into absorbent articles such as diapers. See, for example, US patent 3,699,103 (Harper et al), issued June 13, 1972, and US patent 3,770,731 (Harmon), issued June 20, 1972, that disclose the use of particulate, absorbent, polymeric compositions (often referred to as "hydrogels", "superabsorbents", or "hydrocolloid materials") in absorbent articles.

Conventional particulate, absorbent, polymeric compositions, however, have the limitation that the particles are not immobilised and are free to migrate during processing and/or use. Migration of the particles can lead to material handling losses during manufacturing as well as uncontrolled non-homogeneous distribution of the particles in the structures in which the particles are being used. Especially when the absorbent polymer particles are incorporated in a fibrous matrix at high concentrations, the particles may sift out of the matrix of may become inhomogeneously distributed in an uncontrolled manner, as for instance described in European application number EP-A-0.695.541 (Bogdanski et al.). Another significant problem occurs when these particulate materials migrate during or after swelling in use. Such mobility can lead to high resistance to liquid flow through the material due to the lack of stable interparticle capillary or liquid transport channels. This phenomenon is one form of what is commonly referred to as "gel blocking."

One attempt to overcome the performance limitations associated with absorbent particle mobility during use in absorbent articles is incorporation of the particulate, absorbent, polymeric compositions into tissue laminates, i.e. layered absorbent structures. By encapsulating the particles between tissue layers and affixation of the particles by water bonding or adhesive bonding, the overall particle mobility within an absorbent structure is diminished. However, upon liquid contact, the particles within the laminate are often free to move relative to each other resulting in the breakdown of any pre-existent interparticle capillary channels.

Another attempted solution is to immobilise the particulate, absorbent, polymeric compositions by the addition of large quantities of liquid polyhydroxy compounds that act as an adhesive to hold the particles together or to a substrate, see, for example, US patent 4,410,571 (Korpman), issued October 18, 1983. While this approach does limit migration before and, to some extent, during swelling, the particles eventually become detached from each other or from the substrate in the presence of excess liquid, resulting again in the breakdown of any pre-existing capillary channels between the particles.

Another attempted solution to overcome the problem of absorbent particle mobility is to produce a superabsorbent film by extrusion of a solution of a linear absorbent polymer and subsequently crosslinking it. See, for example, US patent 4,861,539 (Allen et al), issued August 29, 1989 (crosslinked with a polyhydroxy compound such as a glycol or glycerol); and US patent 4,076,673 (Burkholder), issued February 28, 1978 (crosslinked with polyamine-polyamide epichlorohydrin adducts such as Kymene® ). While these superabsorbent films may absorb significant quantities of liquids, they have limited liquid transport properties because they are essentially non-porous, i.e. lack internal capillary channels. Indeed, due to the lack of internal capillary channels, these superabsorbent films are especially prone to gel blocking.

A more recent solution proposed to overcome the problem of absorbent particle mobility is to form these particles into aggregate macrostructures, typically as sheets of bonded absorbent particles. See US patent 5,102,597 (Roe et al), issued April 7, 1992. These aggregate macrostructures are prepared by initially mixing the absorbent particles with a solution of a nonionic crosslinking agent, water and a hydrophilic organic solvent such as isopropanol. These nonionic crosslinking agents include polyhydric alcohols (e.g., glycerol), polyaziridine compounds (e.g., 2,2-bishydroxymethyl butanoltris[3-(1-aziridine) propionate]), haloepoxy compounds (e.g., epicholorhydrin), polyaldehyde compounds (e.g., glutaraldehyde), polyamine compounds (e.g., ethylene amine), and polyisocyanate compounds (e.g., 2,4-toluene diisocyanate), preferably glycerol. See Column 11, lines 22-54, of US patent 5,102,597.

It has been described in US patent 5,102,597 that the hydrogel-forming polymeric precursor particles may be brought into mutual contact by first depositing the particles on a substrate and subsequently effecting inter-particle crosslinking of the contiguous particles.

According to US patent 5,124,188 (Roe et al.), reinforcement structures such as fibers, webs or scrims may be embedded into the macrostructures of crosslinked particles, to provide structural integrity. Embedding the fibers or webs into the macrostructure of crosslinked particles is effected by mixing the fibers with the solution containing the inter-particle crosslinking agent or by mixing the fibers with the particles prior to inter-particle cross-linking. Kneading the fibers into the interparticle crosslinking agent/precursor particle mix is preferred.

In US patent 5,180,622 (Berg), the formation of an inter particle crosslinked aggregate is described, wherein the aggregate is joined to a carrier, which may be comprised of cellulosic fibers or which may be formed by a web. Joining the aggregate and the carrier is generally described as being effected via physical or chemical bonding using adhesives or chemicals that react to adhere the macrostructure, or inter-particle crosslinked aggregate, to the carrier.

The known interparticle crosslinked aggregates are fluid stable as they isotropically expand upon being wetted. Upon wetting of these aggregates, the expansion of the crosslinked polymeric particles generates stress forces which causes the interparticle cross-linked aggregates to bend or curl. This is undesired, especially in absorbent articles, that need to maintain their shape upon becoming wet to effectively absorb discharged body fluids.

One way to avoid the problem of curling and bending of interpaticle crosslinked aggregates, is by providing slits or holes in the aggregates to provide a void space for expansion as described in US-A-5.536.26 (Hsueh). This process requires an additional process step of slitting the aggregates and the substrate to which these aggregates are attached, and optionally expanding the combined substrate and aggregate to form void spaces at the areas of the slits. This process requires that the aggregate is firmly attached to a substrate as the aggregate is often substantially weakened in the slitting and expansion process.

Furthermore, the known interparticle crosslinked aggregates are of uniform structure. In particular applications of crosslinked absorbent aggregates in disposable absorbent articles it is desirable that the absorption properties of the aggregates vary across their surfaces for optimum fluid handling.

It is an object of the present invention to provide a method for making an absorbent structure having distinct liquid-handling properties in different regions.

It is another object of the present invention to provide a method for forming an absorbent structure comprising a plurality of crosslinked polymeric particles in which the problem of curling or bending upon wetting is reduced.

It is another object of the present invention to provide a method for forming an absorbent structure comprising a plurality of crosslinked polymeric particles which has a relatively large structural integrity.

It is a further object of the invention to provide a relatively simple method of making such an absorbent structure.

### SUMMARY OF THE INVENTION

The method according to the invention is characterised in that the crosslinking agent is reacted with the particles in a way such that the degree of crosslinking in the first region differs from the degree of crosslinking in the second region.

With "degree of crosslinking" of the absorbent polymeric particles in a region is meant: a measurable effect of the average number of crosslink bonds between either molecules on the surface of a single particle (surface crosslink bonds) or between molecules of adjacent particles (interparticle crosslink bonds), wherein the average is calculated for the particles in the region, on the liquid handling properties of the region.

The average number of crosslink bonds in a region determines, for absorbent polymeric particles of a pre-determined chemical composition, the liquid-handling properties of the particles in the region. Absorbent polymeric particles having a high number of crosslink bonds on the surface of the particles will swell less rapidly upon absorption of liquids than particles having a lower degree of surface crosslinking. The particles having a high degree of surface crosslinking maintain a relatively open structure when wet and are less liable to gel blocking than particles having a lower degree of surface crosslinking.

When the particles in a particular region comprise a high number of interparticle crosslink bonds, the particles in this region will be firmly attached to one another to form an aggregated macrostructure as for instance described in US Patent 5,124,188 (Roe). When the particles in a region comprise a low degree of interparticle crosslinking, they are less firmly attached and can for instance be removed from these regions of low interparticle crosslinking. However, as upon formation of an interparticle crosslinked aggregate by application of a crosslinking agent, also surface crosslink bonds are formed, the particles in the region of a low degree interparticle crosslinking will tend to also have a low degree of surface crosslinking and will hence have different fluid handling properties than the particles in the region of a high degree of interparticle crosslinking.

When two regions are said to have a different degree of crosslinking, it is meant that after reacting the crosslinking agent with the particles in each region, the chemical and/or physical properties of the particles differ in a way which can be measured by conventional methods. The difference in chemical and/or physical properties may comprise the difference in the degree of swelling of the particles when wetted, the rate of swelling of the particles when wetted, the structural integrity of a number of interpartically crosslinked particles, etc.

The polymeric absorbent particles may for instance be deposited on a tissue and connected to the tissue by adhesive. Subsequently a crosslinking agent may be applied to the adhesively connected particles. Alternatively, the particles may form an aggregated macrostructure to which the crosslinking agent is applied. The aggregated macrostructure may have a high structural integrity as to be self-supporting or may be connected to a reinforcing tissue. Bonding of aggregated macrostructures can effectively be carried out using an interparticle crosslinking agent to form crosslink bonds between the polymeric particles and cellulosic fibers of the tissue as described in US patent US-A-5.536.264.

When a crosslinking agent is applied to the polymeric particles, two types of crosslink bonds can be formed. The first type of crosslink bonds is formed when the crosslinking agent only reacts with the molecules of the polymeric material at the surface of an individual polymeric particle. This type of crosslink bond will be referred to as a "surface crosslink bond".

The second type of crosslink bond is formed when the polymeric particles are contiguous at the moment when the crosslinking reaction takes place, such that neighbouring particles are connected by the crosslinking agent. A bond that is formed between two neighbouring particles by the crosslinking agent will be referred to as an "interparticle crosslink bond".

The physical properties of the layer of polymeric particles are influenced both by the degree of surface crosslinking and by the degree of inter-particle crosslinking of the particles. By locally varying the degree of crosslinking in an absorbent structure, the absorption properties of the absorbent structure can be designed to obtain optimum liquid-handling properties.

The degree of surface crosslinking of the particles will determine the rate with which the particles swell upon absorbing liquids, and will determine the permeability to liquids of a layer comprising surface crosslinked particles.

When the degree of interparticle crosslinking between the particles in an aggregated macrostructure formed by inter-particle crosslinking, is maintained relatively low in a pre-determined region, the particles will adhere less strongly to one another in this region. Hence, the particles which are less strongly attached can after the crosslinking step be removed from the regions in which the particles have a relatively low number of interparticle crosslink bonds. In this way, channels or other patterns of regions having reduced numbers of polymeric particles can be formed. Upon being wetted, the polymeric particles of the aggregated macrostructure can expand into the areas which are devoid of polymeric particles or which comprise a relatively low number of polymeric particles. The problem of bending or curling of the aggregated macrostructure is hereby reduced.

It is however also possible that the polymeric particles are not removed from the regions having a low degree of interparticle crosslinking. Different degrees of surface crosslinking and/or interparticle crosslinking in an aggregated macrostructure, results in locally varying liquid handling properties even though the density and basis weight of polymeric particles are homogeneous across the regions.

The degree of crosslinking of the absorbent polymeric particles in a region is determined by the crosslinking conditions upon formation of the crosslink bonds. The degree of crosslinking will depend for instance on:
- the amount of crosslinking agent applied,
- the chemical composition of the polymeric particles and crosslinking agent,
- the duration of the crosslinking reaction,
- the temperature and pressure during formation of the crosslink bonds, and
- the interparticle spacing upon forming the crosslink bonds. This will determine if surface crosslink bonds or interparticle crosslink bonds are formed.

The degree of crosslinking of the polymeric particles can be varied in a number of ways. Firstly, the amount of crosslinking agent applied to the polymeric particles can be varied across the surface of the absorbent structure. The number of crosslink bonds that is formed upon application of the crosslinking agent will depend on the concentration of crosslinking agent that is applied to the polymeric particles.

The amount of crosslinking agent that is applied may be varied by uniformly laying down the particles on a conveyor or tissue and forwarding them underneath an applicator of crosslinking agent. The applicator can be a spray nozzle that emits the crosslinking agent according to a predetermined pattern.

The pattern of crosslinking agent may be obtained by placing a mask between the applicator of crosslinking agent and the particles. The mask can comprise relatively open areas which readily transmit the cross-linking agent and closed areas which block the crosslinking agent.

Alternatively, the applicator of crosslinking agent is operated in an intermittent manner such that the amount of crosslinking agent that is emitted by the applicator varies in time when the particles are transported below the applicator. Again, alternatively, the particles may be transported below a multiplicity of applicators applying different amounts of crosslinking agent may be located over the particles.

Secondly, the degree of crosslinking can be varied by applying a crosslinking agent of different chemical composition to different regions of the absorbent structure. The affinity to form surface crosslink bonds may for instance be large for one crosslinking agent, whereas the affinity for forming inter-particle crosslink bonds is larger for another crosslinking agent. Alternatively, the rate of formation of crosslink bonds may be different for the different crosslinking agents at similar reaction conditions.

Thirdly, the degree of crosslinking depends on the chemical composition and on the physical properties of the polymeric particles that are used. The physical properties of the particles include the particle size distribution, the average particle size and the particle shape (fibers of spheres). The chemical properties include the extent of crosslinking of the monomer material from which the particles are formed and the extent of surface crosslinking of the particles that is already present before application of the crosslinking agent.

Fourthly, the reaction conditions across the surface of the absorbent structure can be locally varied to obtain different degrees of crosslinking in different regions while crosslinking agent is applied homogeneously to the polymeric particles. This can be achieved by uniformly applying the crosslinking agent, and by subsequently curing the polymeric particles by application of ionising radiation, such as by application of visible - or UV light, electron beam radiation, x-rays or a laser. The ionising radiation, which generates free radicals that cause the inter-particle cross linking reaction, is applied in a non-uniform manner across the absorbent structure. A mask may be placed between the source of ionising radiation and the particles, the mask having areas of varying transparency for the radiation. When the mask is projected onto the particles by the ionising radiation, the number of crosslink bonds formed will be relatively high in the transparent areas of the mask.

Alternatively, when a laser or an electron beam is used as a source of ionising radiation, the electron or laser beam may be moved across the particles is a specific pattern. Polymerisation reactions wherein polymerisation is induced by ionising radiation are disclosed in US patent no. 4,646,730 (Schonfeld) and EP-A-0 054 841 (Bloch).

Alternatively, the rate at which crosslinking reactions take place or the duration thereof may be varied by locally influencing heat and pressure conditions, for instance by passing the particles through a patterned heated pressure nip.

Finally, the degree of interparticle crosslinking can be influenced by adapting the average spacing of the polymeric particles. When the polymeric particles are spaced apart, less interparticle crosslinking will occur than when the polymeric particles are compacted. Therefore, another way to vary the degree of crosslinking in different regions is by non-uniform deposition of the particles in these regions, such that regions of high and lower density and/or basis weights of particles are formed. In the areas of lower density and/or basis weights the inter-particle distance will be relatively large, such that in these areas less inter-particle crosslink bonds are formed compared to areas where the particles are closer together.

Prior to initiating the crosslinking reaction between the polymeric particles and the crosslinking agent, the particles can have a number of configurations:

The particles may be agglomerated to form an aggregated macrostructure by means of moisture, such as steam, or by means of inter-particle crosslinking agent as described in US patent no. 5,102,597, or EP Application EP-A-0.662.848.

Alternatively, the particles may be connected to a tissue layer by means of adhesive or by hydrogen bonding to form an absorbent structure.

Again, alternatively, the particles may be laid down on a conveyor or on a tissue in a contiguous manner, such that upon crosslinking of the particles, inter-particle crosslink bonds are formed.

In each case, the polymeric particles may, before effecting the crosslinking reaction according to the invention, have a pre-determined degree of inter-particle crosslinking or surface crosslinking. Surface cross-linking of polymeric absorbent particles is described in EP application EP-A-0.662.848.

### BRIEF DESCRIPTION OF THE DRAWINGS

The invention will be described in detail with reference to the accompanying drawings. In the drawings:
Figure 1 shows a cross-sectional view of a known interparticle crosslinked aggregate,
Figure 2 and 3 show a cross-sectional view of an interparticle crosslinked aggregate according to the invention,
Figure 4 schematically shows a production line for manufacturing an absorbent structure according to the invention,
Figure 4a show an enlarged detail from figure 4,
Figures 5a-5c show photographs of an absorbent structure wherein the absorbent particles have been removed from the regions of low degrees of cross-linking,
Figures 6a-6c show photographs of an absorbent structure comprising regions of highly crosslinked absorbent particles and regions where the absorbent particles have a relatively low degree of crosslinking,
Figures 7-11 show different methods of manufacturing an absorbent structure according to the invention,
Figures 12a, 12b show side elevational views of a patterned compression roll,
Figures 13-15 show further embodiments of methods of manufacturing an absorbent article according to the invention,
Figures 16-20 show cross-sectional views of absorbent structures according to the invention,
Figures 21-26 show plan views of absorbent structures according to the invention, and
Figures 27 and 28 show plan views of masks for use in the manufacture of absorbent structures.

### DETAILED DESCRIPTION OF THE INVENTION

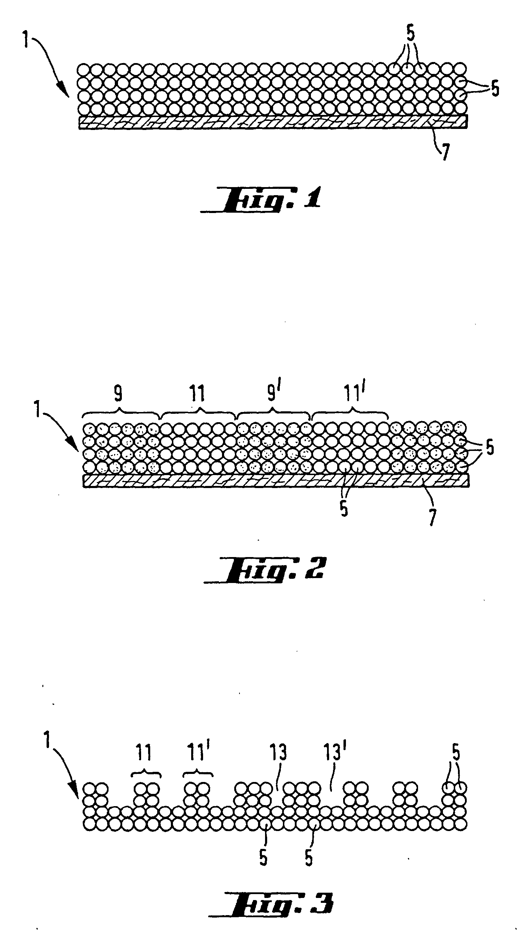

Figure 1 shows an interparticle crosslinked aggregate 1 as described in for instance US patent No 5,124,188 (Roe). The aggregate comprises polymeric particles 5 that can have a size between 50 and 1000 micrometers. The polymeric particles 5 are mutually connected by interparticle crosslink bonds, such that an agglomerated macrostructure or aggregate 1 is formed. The crosslinked aggregate can be self-supporting or may be connected to a substrate 7 by means of gluing, or chemical bonding. A preferred way of attaching the aggregate 1 to the substrate 7 is described in US-A-5.536.26 (Hsueh) and comprises the step of crosslinking the particles 5 to the substrate 7 by means of the interparticle crosslinking agent that also interconnects adjacent polymeric particles.

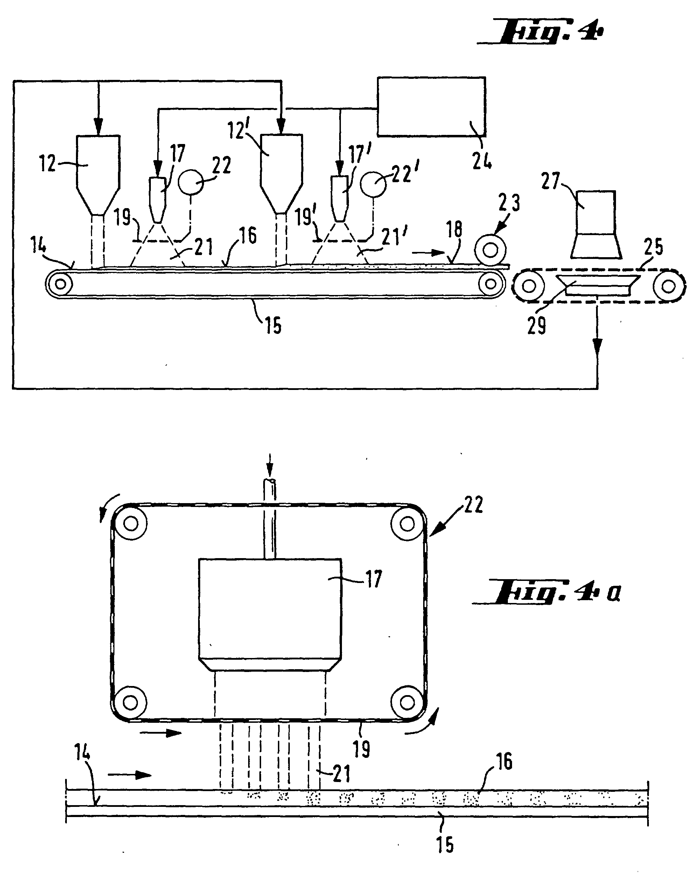

Figure 2 shows an interparticle crosslinked aggregate 1 according to the invention, comprising regions 9,9' in which the degree of crosslinking of the individual particles 5 is less than in the regions 11, 11'. Hence the rate of liquid absorption in the regions 9 and 11 will differ, and the amount of swelling of the particles 5 in the different regions 9,11 will vary. Preferably the volume of the regions 9,9', 11,11' is at least 10 mm³.

In figure 3, it is shown that the particles in regions 13,13' of the lower degree of crosslinking have been removed to form open spaces which allow expansion of the particles 5 without the shape of the crosslinked aggregate 1 being negatively affected by bending or curling.

Figure 4 shows the equipment for forming interparticle crosslinked aggregates as shown in figure 2 or 3. The particles are deposited from an applicator such as a chute, or powder spray gun 12 onto a conveyor belt 15 which forms a deposition surface 14. The particles are laid down in a uniform layer 16 and are passed below a spray nozzle 17 which applies crosslinking agent, supplied from a container 24, to the mutually contiguous particles. The layer 16 may be laid down by pulsed or intermittent operation of the powder spray gun. The spray of crosslinking agent 21 that is emitted by the nozzle 17 is divided into a number of jets by a mask 19. Each jet that passes through the mask 19 impinges upon a region of the deposition surface such that a non-uniform application of the crosslinking agent across the deposition surface is achieved. At least one further powder spray gun 12' and nozzle 17' may be located downstream of the spray gun 12 and nozzle 17, to deposit additional layers of particles onto the layer 16. To obtain varying crosslinking patterns in the direction of transport of the conveyor belt 15, the conveyor belt 15 may be periodically stopped and may be synchronized with the application of crosslinking agent from the spray nozzles 17, 17'. A suitable mechanism for periodically stopping a part of a continuously moving conveyor belt is described in PCT publication WO 95/12539.

Alternatively, translating means 22 are provided for translating the mask 19 in the direction of transport of the conveyor 15, to allow a stationary projection of the mask 19 onto the deposition surface 14 while continuously moving the conveyor belt 15, as shown in figure 4a.

After passing through a compression nip 23, the crosslinked aggregate 18 is passed onto a foraminous belt 25 which passes between an airgun 27 and a suction box 29. The polymeric particles in the aggregate 18 to which no crosslinking agent has been applied are removed from the layer 16 by the airgun and are collected by the suction box 29. The removed particles are reapplied to the powder spray guns 12, 12'.

### The interparticle crosslinked aggregate, or macrostructure

Porous, absorbent macrostructures used in the absorbent composites according to the present invention are structures capable of absorbing large quantities of liquids such as water and/or body exudates (e.g., urine or menses) and then retaining such liquids under moderate pressures. Because of the particulate nature of the absorbent polymeric particles from which the macrostructures are formed, or precursor particles, the macrostructure has pores between adjacent precursor particles. These pores are interconnected by intercommunicating channels such that the macrostructure is liquid permeable (i.e., has capillary transport channels).

Due to the crosslinking of the absorbent polymers in the interconnected surface portions of adjacent, interconnected polymeric particles, the resultant absorbent macrostructure has good structural integrity, increased liquid acquisition and distribution rates, and minimal gel-blocking characteristics. When contacted with liquid, the absorbent macrostructure absorbs such liquids into the pores between the precursor particles, and then imbibes such liquids into the particles, whereby the absorbent macrostructure swells generally isotropically even under moderate confining pressures. Isotropic swelling is used herein to mean that the macrostructure swells generally equally in all directions when wetted. Isotropic swelling is an important property of the macrostructure because the absorbent gelling particles and their associated pores are able to maintain their relative geometry and spatial relationships even when swollen, such that the existing capillary channels are maintained, if not enlarged, during use. (The pores and the absorbent gelling particles get larger during swelling.) Thus, the macrostructure can imbibe and/or transport through itself additional loadings of liquid while not gel blocking. Upon swelling, the particles can expand into the zones of relatively little crosslinking such that the stress forces that cause bending and curling in the known interparticle crosslinked macrostructures, are relieved.

An indication that crosslink bonds are being formed at the surface of the absorbent polymeric particles (hereinafter also referred to as precursor particles) is that the resultant macrostructures are fluid (i.e., liquid) stable. "Fluid stable" is used herein to mean a macrostructure comprising an aggregate of interconnected particles that remains substantially intact (i.e., most of the previously independent component precursor particles remain bonded together) upon contact with or swelling (with and/or without stress) in an aqueous fluid. While this definition of fluid stability recognises that most, preferably all, of the precursor particles remain bonded together, some of the precursor particles can dissociate themselves from the macrostructure if, for example, other particles have been subsequently water agglomerated onto it.

Fluid stability is an important feature of the absorbent macrostructure layers in the present invention because it allows the aggregate to maintain its relative structure in both the dry and swollen states, and because it immobilises component precursor particles. In an end product such as an absorbent member or an absorbent article, fluid stability is beneficial in reducing gel blocking since precursor particles remain aggregated even when contacted with liquid, and allows one to use previously independent fine particles in an aggregate form to increase the rate of fluid uptake of the resultant macrostructure without introducing the element of gel blocking.

Fluid stability can be measured in an aggregate macrostructure by a two step process. The initial dynamic response of the aggregate macrostructure upon contact with the aqueous fluid is observed and then the fully swollen equilibrium condition of the aggregate macrostructure is observed. A test method for determining fluid stability based on these criteria is described in detail in US patent no. 5,124,188 (Roe et al.) on Column 28 line 51 to Column 29 line 4.

As used herein, the term "macrostructure" means a structure having a circumscribed volume when substantially dry (i.e., circumscribed dry volume) of at least about 10.0 mm³, more preferably at least about 100 mm³, most preferably at least about 500 mm³. Typically, the macrostructures of the present invention will have a circumscribed dry volume much greater than about 500 mm³. In preferred embodiments of the present invention, the macrostructures have a circumscribed dry volume of between about 1000 mm³ and about 100,000 mm³.

While the macrostructures used in absorbent composites of the present invention can have a number of shapes and sizes, they are typically in the form of sheets, films, cylinders, blocks, spheres, fibers, filaments, or other shaped elements. The macrostructures will generally have a thickness or diameter between about 0.2 mm and about 10.0 mm. Preferably for use in absorbent products, the macrostructures are in the form of a sheet. The term "sheet" as used herein describes macrostructures having a thickness at least about 0.2 mm. The sheets will preferably have a thickness between about 0.5 mm and about 10 mm, typically from about 1 mm to about 3 mm.

The porous, absorbent macrostructures used in the absorbent composite of the present invention comprise aggregates of interconnected particles. These aggregates of interconnected particles usually comprise about 8 or more previously independent precursor particles. For preferred circumscribed dry volumes and sizes of the individual precursor particles used herein, these aggregates of interconnected particles typically are formed from about 100,000 or more individual precursor particles. These individual precursor particles can comprise granules, pulverulents, spheres, flakes, fibers, aggregates or agglomerates.

The individual precursor particles can have a variety of shapes, such as cubic, rod-like, polyhedral, spherical, rounded, angular, irregular, porous-on-surface, randomly-sized irregular shapes, e.g., pulverulent products of grinding or pulverising steps, or shapes having a large greatest dimension/smallest dimension ratio so as to be needle-like, plate-like, flake-like, or fiber-like. An example of porous-on-surface precursor particles is disclosed in US patent 5,118,719, issued June 2, 1992, to which particular reference is made.

The aggregate of interconnected particles comprising the macrostructures of the present invention are formed, in essence, by connecting together of adjacent particles. The interconnection of adjacent particles is essentially made by the polymeric material that is present in the surface portions of these particles. Treatment of the precursor particles comprises contacting portions of their surfaces with a crosslinking agent, and a swelling agent, preferably water. In preferred embodiments, a plasticizer is also used, most preferably in combination with the treatment agent, to improve the flexibility and integrity of the porous absorbent macrostructure. When these precursor particles are treated at portions of the surface of the particles, and physically associated as described hereafter, the polymer material present in the surface portions of these particles becomes sufficiently plastic (softened) and cohesive (e.g., sticky) such that adjacent particles cohesively adhere together. When the crosslinking agent reacts with the polymer material of the interconnected surface portions of the particles, the interconnected portions become set, strong, and fast absorbing, thereby forming the porous absorbent macrostructure.

The quantity of the absorbent particles comprised in the absorbent macrostructure layer, or the thickness of the sheet of the absorbent macrostructure layer, can be varied to provide the absorbent composite with different amounts of absorbency. In preferred embodiments of an absorbent composite in the forms of a sheet, the basis weight of an absorbent macrostructure layer (expressed as weight of absorbent macrostructure per unit area of the absorbent macrostructure layer) is from about 100 g/m² to about 1500 g/m² in average, more preferably from about 250 g/m² to about 1200 g/m² in average. In addition, the absorbent macrostructure layer preferably has a density of from about 0.6 g/cc to about 1.1 g/cc.

The percent void volume (i.e., the percent of volume of the macrostructure that comprises the pores and the channels) has a minimum value for a given precursor particle size distribution. In general, when the precursor particle size distribution is more narrow, the percent volume void is higher. Thus, it is preferred, so that the precursor particles have a relatively narrow particle size distribution, to provide a higher percent volume void in a densified state. Also, in general, when the precursor particle size is larger, the permeability increases.

The absorbent macrostructure can also comprise a plurality of absorbent gelling particle layers each comprising absorbent gelling particles. The plurality of absorbent gelling particle layers can have substantially different particle sizes at least in two adjacent particle layers. Preferably, the mass average particle sizes of the plurality of absorbent gelling particle layers are changed with successive particle layers. For instance, the absorbent macrostructure may comprise a plurality of layers of absorbent gelling particles where the mass average particle size of the layered particles is gradually reduced in the direction away from the substrate layer 7. On the other hand, the macrostructure may comprise a plurality of layers of absorbent gelling particles where the mass average particle size of the layered particles is gradually increased in the direction away from the substrate layer 7. It is believed that the layering of particles of increasing (or decreasing) particle size in the porous macrostructures can provide benefits in fluid acquisition, distribution and storage. For example, in a macrostructure layer which has upper most particle layers with particles relatively larger than those in the lower most layers, the larger void areas between adjacent larger particles in the upper layers have a relatively greater porosity and fluency for the liquids, allowing a substantial portion of the fluid to pass therethrough to the lower layers. In the lower layers, because of the smaller pores and void spaces, the liquids are readily acquired and absorbed into the smaller absorbent particles, which then begin to swell. The larger particles in the upper most layer remain available to acquire and absorb additional deposits of liquid. Particularly in the case of absorbent diapers, where multiple depositions of liquids are expected, the layering of particles by size can optimize the absorbent capacity of the absorbent material.

In a similar manner, an absorbent macrostructure can comprise a plurality of particle layers wherein the absorbent polymeric particles of any two layers can have different absorbent gelling particle properties. These different absorbent polymeric particle properties can include, for example, different liquid absorption rate, different liquid absorption capacity, different particle shape, different particle gel strength, or combinations of these different properties. As in the case of absorbent gelling particle size, the successive layers of absorbent gelling particles can be ordered in terms of these absorbent gelling particle properties. By way of example, an absorbent macrostructure can be made by having faster absorbent gelling particles in the bottom-most layers, adjacent to the substrate, and slower absorbent gelling particles near the surface.

In an alternate embodiment, layers of absorbent polymeric particles can be separated by a layer of non-absorbent, non-gelling material, preferably in particulate form. Such non-absorbent, non-gelling material can include absorbent fibers or other forms as described hereinafter, or can include particulate material which can provide other functions, such as odor control.

The absorbent macrostructure layer can optionally comprise non-absorbent, non-gelling materials, such as fibers. Such fiber material can be used as reinforcing members in the macrostructure layers of the present invention, as well as a co-absorbent with the absorbent gelling particles. Any type of fiber material which is suitable for use in conventional absorbent products can be used in the macrostructures herein. Specific examples of such fiber material include cellulose fibers, modified cellulose fibers, rayon, polypropylene, and polyester fibers such as polyethylene terephthalate (DACRON), hydrophilic nylon (HYDROFIL), and the like. Examples of other fiber materials for use in the present invention in addition to some already discussed are hydrophilized hydrophobic fibers, such as surfactant-treated or silica-treated thermoplastic fibers derived, for example, from polyolefins such as polyethylene or polypropylene, polyacrylics, polyamides, polystyrenes, polyurethanes and the like. In fact, hydrophilized hydrophobic fibers which are in and of themselves not very absorbent and which, therefore, do not provide webs of sufficient absorbent capacity to be useful in conventional absorbent structures, are suitable for use in the macrostructure layers of the present invention by virtue of their good wicking properties. This is because, in the macrostructures herein, the wicking propensity of the fibers is as important, if not more important, than the absorbent capacity of the fiber material itself due to the high rate of fluid uptake and lack of gel blocking properties of the macrostructure layers in the present invention. Synthetic fibers are generally preferred for use herein as the fiber component of the macrostructure layers. Most preferred are polyolefin fibers, preferably polyethylene fibers.

Other cellulosic fiber materials which can be useful in certain macrostructure layers herein are chemically stiffened cellulosic fibers. Preferred chemically stiffened cellulosic fibers are the stiffened, twisted, curled cellulosic fibers which can be produced by internally crosslinking cellulose fibers with a crosslinking agent. Suitable stiffened, twisted, curled cellulose fibers useful as the hydrophilic fiber material herein are described in greater detail in US patent 4,888,093 (Dean et al), issued December 19, 1989; US patent 4,889,595 (Herron et al), issued December 26, 1989; US patent 4,889,596 (Schoggen et al), issued December 26, 1989; US patent 4,889,597 (Bourbon et al), issued December 26, 1989; and US patent 4,898,647 (Moore et al), issued February 6, 1990.

As used herein, the term "hydrophilic" describes fibers or the surfaces of fibers which are wetted by the aqueous liquids deposited onto the fibers (i.e., if water or aqueous body fluid readily spreads on or over the surface of the fiber without regard to whether or not the fiber actually imbibes fluid or forms a gel). The state of the art respecting wetting of materials allows definition of hydrophobicity (and wetting) in terms of contact angles and the surface tension of the liquids and solids involved. This is discussed in detail in the American Chemical Society Publication entitled "Contact Angle, Wettability, and Adhesion edited by Robert F. Gould and copyrigthed in 1964. A fiber or surface of a fiber is said to be wetted by a liquid either when the contact angle between the liquid and the fiber or surface is less than 90° or when the liquid will tend to spread spontaneously across the surface of the fiber; both conditions are normally coexisting.

Other materials to provide various additional functionality. Such additional functionality can include porosity, permeability, odor control, wetness indication, structural flexibility, and structural integrity. Non-limiting examples of such other materials include : filler material, such as silica; wetness indicators; and oder control agents such as those disclosed in US patent 5,161,686.

In another preferred embodiment, the absorbent macrostructure layer can optionally comprise foam particles (or granules) mixed with the absorbent gelling particles, such a cellulose foam particles. Preferably, the cellulose foam particles have an average volume of at least about 0.1 mm³, more preferably from about 1.0 mm³ to about 125 mm³.

The absorbent macrostructure layer typically comprises from about 40% to about 100%, preferably from about 50% to about 100%, and more preferably about 70% or more by weight of absorbent gelling particles.

### Absorbent Precursor Particles

The macrostructures used in the present invention are formed from polymer materials capable of absorbing large quantities of liquids. (Such polymer materials are commonly referred to as "hydrogel", "hydrocolloid", or "superabsorbent" materials.) The macrostructures preferably comprise substantially water-insoluble, absorbent hydrogel-forming, polymer material. The specific polymer materials will be discussed herein with respect to those forming the absorbent gelling particles (hereinafter also referred to as "precursor particles").

Although the precursor particles can have a size varying over a wide range, specific particle size distributions and sizes are preferred. For purposes of the present invention, particle size is defined for precursor particles that do not have a large greatest dimension/smallest dimension ratio such as fibers (e.g., granules, flakes, or pulverulents) as the dimension of a precursor particle which is determined by sieve size analysis. Thus, for example, a precursor particle that is retained on a standard #30 sieve with 600 micron openings is considered to have a particle size greater than 600 microns, a precursor particle that passes through the #30 sieve with 600 micron openings and is retained on a standard #35 sieve with 500 micron openings is considered to have a particle size between 500 and 600 microns, and a precursor particle that passes through a #35 sieve with 500 micron openings is considered to have a particle size less than 500 microns. In preferred embodiments of the present invention, the precursor particles will generally range in size from about 1 micron to about 2000 microns, more preferably from about 20 microns to about 1000 microns.

Further, for purposes of this invention, the mass average particle size of the precursor particles is important in determining the characteristics and properties of the resultant macrostructures. The mass average particle size of a given sample of precursor particles is defined as the particle size which is the average particle size of the sample on a mass basis. A method for determining the mass average particle size of a sample is described in detail in US patent no. 5,124,188 (Roe et al.) on Column 29 line 5 to Column 30 line 4. The mass average particle size of the precursor particles will generally be from about 20 microns to about 1500 microns, more preferably from about 50 microns to about 1000 microns, most preferably from about 50 microns to about 800 microns. In especially preferred embodiments, the mass average particle sizes is from about 100 microns to about 250 microns. The particles can be substantially uniform in size and shape, or can be randomly or ordered in size and shape. In an exemplary embodiment, at least about 95% by weight of the precursor particles have a particle size between about 150 microns and about 300 microns. In an alternative embodiment, at least about 95% by weight of the precursor particles have a particle size between about 90 microns and about 180 microns. Narrow precursor particle size distributions are preferred because they result in a higher porosity macrostructure due to the higher void fraction when densified versus broader precursor particle size distributions with equivalent mass average particle sizes. However, wider particle size distributions can result in improved integrity of the macrostructures by providing an increased number of bonding points per unit volume.

The particle size of materials having a large greatest dimension/smallest dimension such as fibers is typically defined by their largest dimension. For example, if absorbent, polymeric fibers (i.e. superabsorbent fibers) are used in the macrostructures, the length of the fibers is used to define the "particle size" (The diameter of the fibers can also be specified, e.g. expressed in denier). In exemplary embodiments of the present invention, the fibers have a length greater than about 5 mm, preferably between about 10 mm and about 100 mm, more preferably between about 10 mm and about 50 mm.

The precursor particles comprise substantially water-insoluble, absorbent hydrogel-forming, polymer material having a multiplicity of anionic, functional groups, such as sulfonic acid, and more typically carboxy, groups. Examples of polymer materials suitable for use as the precursor particles herein include those which are prepared from polymerizable, unsaturated, acid-containing monomers. Thus, such monomers include the olefinically unsaturated acids and anhydrides which contain at least one carbon to carbon olefinic double bond. More specifically, these monomers can be selected from olefinically unsaturated carboxylic acids and acid anhydrides, olefinically unsaturated sulfonic acids, and mixtures thereof.

Some non-acid monomers can also be included, usually in minor amounts, in preparing the precursor particles herein. Such non-acid monomers can include, for example, the water-soluble or water-dispersible esters of the acid-containing monomers, as well as monomers which contain no carboxylic or sulfonic acid groups at all. Optional non-acid monomers can thus include monomers containing the following types of functional groups: carboxylic acid or sulfonic acid esters, hydroxyl groups, amide-groups, amino groups, nitrile groups and quaternary ammomium salt groups. These non-acid monomers are well-known materials and are described in greater detail, for example, in US patent 4,076,663 (Masuda et al), issued February 28, 1978, and in US patent 4,062,817 (Westerman), issued December 13, 1977, to both of which particular reference is made.

Olefinically unsaturated carboxylic acid and carboxylic acid anhydride monomers include the acrylic acids typified by acrylic acid itself, methacrylic acid, ethacrylic acid, a-chloroacrylic acid, a-cyanoacrylic acid, b-methylacrylic acid (crotonic acid), a-phenylacrylic acid, b-acryloxypropionic acid, sorbic acid, a-chlorosorbic acid, angelic acid, cinnamic acid, p-chloro cinnamic acid, b-sterylacrylic acid, itaconic acid, citroconic acid, mesaconic acid, glutaconic acid, aconitic acid, maleic acid, fumaric acid, tricarboxyethylene and maleic acid anhydride.

Olefinically unsaturated sulfonic acid monomers include aliphatic or aromatic vinyl sulfonic acids such as vinylsulfonic acid, allyl sulfonic acid, vinyltoluene sulfonic acid and styrene sulfonic acid; acrylic and methacrylic sulfonic acid such as sulfoethyl acrylate, sulfoethyl methacrylate, sulfopropyl acrylate, sulfopropyl methacrylate, 2-hydroxy-3-methacryloxy propyl sulfonic acid and 2-acrylamide-2-methylpropane sulfonic acid.

Preferred polymer materials for use in the present invention contain carboxy groups. These polymers include hydrolyzed starch-acrylonitrile graft copolymers, partially neutralised starch-acrylonitrile graft copolymers, starch acrylic acid graft copolymers, partially neutralised starch-acrylic acid graft copolymers, saponified vinyl acetate-acrylic ester copolymers, hydrolysed acrylonitrile or acrylamide copolymers, slightly network crosslinked polymers of any of the foregoing copolymers, partially neutralized polyacrylic acid, and slightly network crosslinked polymers of partially neutralized polyacrylic acid. These polymers can be used either solely or in the form of a mixture of two or more different polymers. Examples of these polymer materials are disclosed in US patent 3,661,875, US patent 4,076,663, US patent 4,093,776, US patent 4,666,983, and US patent 4,734,478.

Most preferred polymer materials for use in making the precursor particles are slightly network crosslinked polymers of partially neutralised polyacrylic acids and starch derivatives thereof. Most preferably, the precursor particles comprise from about 50% to about 95%, preferably about 75%, neutralised, slightly network crosslinked, polyacrylic acid (i.e. poly (sodium acrylate/acrylic acid)). As described above, the precursor particles are preferably made from polymer materials that are slightly network crosslinked. Network crosslinking serves to render the polymer materials from which the precursor particles are made substantially water-insoluble and, in part, determines the absorptive capacity and extractable polymer content characteristics of the precursor particles and the resultant macrostructures. Processes for network crosslinking the polymers and typical network crosslinking agents are described in greater detail in the hereinbefore-referenced US patent 4,076,663.

The individual precursor particles can be formed in any conventional manner. Typical and preferred processes for producing the individual precursor particles are described in US patent Re. 32,649 (Brandt et al), issued April 19, 1988, US patent 4,666,983 (Tsubakimoto et al), issued May 19, 1987, and US patent 4,625,001 (Tsubakimoto et al), issued November 25, 1986, to all of which particular reference is made. Preferred methods for forming the precursor particles are those that involve aqueous solution or other solution polymerization methods. As described in the above-referenced US patent Re. 32,649, aqueous solution polymerization involves the use of an aqueous reaction mixture to carry out polymerization to form the precursor particles. The aqueous reaction mixture is then subjected to polymerization conditions which are sufficient to produce in the mixture, substantially water-insoluble, slightly network crosslinked polymer material. The mass of polymer material thereby formed is then pulverized or chopped to form the individual precursor particles.

More specifically, the aqueous solution polymerization method for producing the individual precursor particles comprises the preparation of an aqueous reaction mixture in which to carry out polymerization to form the desired precursor particles. One element of such a reaction mixture is the acid group-containing monomer material which will form the "backbone" of the precursor particles to be produced. The reaction mixture will generally comprise about 100 parts by weight of the monomer material. Another component of the aqueous reaction mixture comprises a network crosslinking agent. Network crosslinking agents useful in forming the precursor particles are described in more detail in the above-referenced US patent Re. 32,649, US patent 4,666,983, and US patent 4,625,001. The network crosslinking agent will generally be present in the aqueous reaction mixture in an amount of from about 0.001 mole percent to about 5 mole percent based on the total moles of monomer present in the aqueous mixture (about 0.01 to about 20 parts by weight, based on 100 parts by weight of the monomer material). An optional component of the aqueous reaction mixture comprises a free radical initiator including, for example, peroxygen compounds such as sodium, potassium, and ammonium persulfates, caprylyl peroxide, benzoyl peroxide, hydrogen peroxide, cumene hydroperoxides, tertiary butyl diperphthalate, tertiary butyl perbenzoate, sodium peracetate, sodium percarbonate, and the like. Other optional components of the aqueous reaction mixture comprise the various non-acidic comonomer materials including esters of the essential unsaturated acidic functional group-containing monomers or other comonomers containing no carboxylic or sulfonic acid functionalities at all.

The aqueous reaction mixture is subjected to polymerization conditions which are sufficient to produce in the mixture substantially water-insoluble, absorbent, hydrogel-forming, slightly network crosslinked polymer materials. The polymerization conditions are also discussed in more detail in the three above-referenced patents. Such polymerization conditions generally involve heating (thermal activation techniques) to a polymerization temperature from about 0°C to about 100°C , more preferably from about 5°C to about 40°C . Polymerization conditions under which the aqueous reaction mixture is maintained can also include, for example, subjecting the reaction mixture, or portions thereof, to any conventional form of polymerization activating irradiation. Radioactive, electronic, ultraviolet, or electromagnetic radiation are alternative conventional polymerization techniques.

The acid functional groups of the polymer materials formed in the aqueous reaction mixture are also preferably neutralized. Neutralization can be carried out in any conventional manner which results in at least about 25 mole percent, and more preferably at least about 50 mole percent, of the total monomer utilized to form the polymer material being acid group-containing monomers that are neutralized with a salt-forming cation. Such salt-forming cations include, for example, alkali metals, ammonium, substituted ammonium and amines as discussed in further detail in the above mentioned US patent Re. 32,649. While it is preferred that the precursor particles be manufactured using an aqueous solution polymerization process, it is also possible to carry out the polymerization process using multi-phase polymerization processing techniques such as inverse emulsion polymerization or inverse suspension polymerization procedures. In the inverse emulsion polymerization or inverse suspension polymerization procedures, the aqueous reaction mixture as hereinbefore described is suspended in the form of tiny droplets in a matrix of a water-immiscible, inert organic solvent such as cyclohexane. The resultant precursor particles are generally spherical in shape. Inverse suspension polymerization procedures are described in greater detail in US patent 4,340,706 (Obaysashi et al), issued July 20, 1982, US patent 4,506,052 (Flesher et al), issued March 19, 1985, and US patent 4,735,987 (Morita et al), issued April 5, 1988, to all of which particular reference is made.

The precursor particles are preferably substantially dry. The term "substantially dry" is used herein to mean that the precursor particles have a liquid content, typically water or other solution content, less than about 50%, preferably less than about 20%, more preferably less than about 10%, by weight of the precursor particles. Most preferably, the liquid content of the precursor particles is in the range of from about 0.01% to about 5% by weight of the precursor particles. The individual precursor particles can be dried by any conventional method such as by heating. Alternatively, when the precursor particles are formed using an aqueous reaction mixture, water can be removed from the reaction mixture by azeotropic distillation. The polymer-containing aqueous reaction mixture can also be treated with a dewatering solvent such as methanol. Combinations of these drying procedures can also be used. The dewatered mass of polymer material can then be chopped or pulverized to form substantially dry precursor particles of substantially water-insoluble, absorbent, hydrogel-forming, polymer material.

Preferred precursor particles of the present invention are those which exhibit a high absorptive capacity so that the resultant macrostructure formed from such precursor particles also has a high absorptive capacity. Absorptive capacity refers to the capacity of a given polymer material to absorb liquids with which it comes into contact. Absorptive capacity can vary significantly with the nature of the liquid being absorbed and with the manner in which the liquid contacts the polymer material. Absorptive Capacity is defined in terms of the amount of Synthetic Urine absorbed by any given polymer material in terms of grams of Synthetic Urine per gram of polymer material under conditions as specified in the Teabag Centrifuge Capacity test such as specified in WO 95/01147. Preferred precursor particles of the present invention are those which have an Absorptive Capacity of at least about 20 grams, more preferably at least about 25 grams, of Synthetic Urine per gram of polymer material. Typically, the polymer materials of the precursor particles herein have an Absorptive Capacity of from about 20 grams to about 70 grams of Synthetic Urine per gram of polymer material. Precursor particles having this relatively high absorptive capacity characteristic produce macrostructures that are especially useful in absorbent products, absorbent members, and absorbent articles since the resultant macrostructures formed from such precursor particles can, by definition, hold desirably high amounts of discharged body exudates such as urine.

While all of the precursor particles are preferably formed from the same polymer material with the same properties, this need not be the case. For example, some precursor particles can comprise a starch-acrylic acid graft copolymer while other precursor particles can comprise a slightly network crosslinked polymer of partially neutralized polyacrylic acid. Further, the precursor particles can vary in size, shape, absorptive capacity, or any other property or characteristic. In a preferred embodiment of the present invention, the precursor particles consist essentially of slightly network crosslinked polymers of partially neutralized polyacrylic acid, each precursor particle having similar properties.

In another embodiment of the present invention, the precursor particles can themselves be crosslinked at least at a portion of, preferably substantially all of, their surfaces, prior to forming the precursor particles into an absorbent macrostructure. The surface crosslinking of precursor particles can be made by any of the crosslinking agents described hereinafter. Preferred crosslinking agents preferably have relatively large molecular size, and are preferably cationic. Such a crosslinking agent is unable to penetrate inside the absorbent particles, and therefore can only react with polymer material at the surface thereof effectively. Most preferably, the crosslinking agent is a cationic amino-epichlorohydrin adduct.

Surface crosslinked hydrogel-forming absorbent polymers have a higher level of crosslinking in the vicinity of the surface than in the interior. As used herein, "surface" describes the outer-facing boundaries of the particle, fiber, etc. For porous hydrogel-forming absorbent polymers (e.g., porous particles, etc.), exposed internal boundaries can also be included. By a higher level of crosslinking at the surface, it is meant that the level of functional crosslinks for the hydrogel-forming absorbent polymer in the vicinity of the surface is generally higher than the level of functional crosslinks for the polymer in the interior.

The gradation in crosslinking from surface to interior can vary, both in depth and profile. Thus, for example, the depth of surface crosslinking can be shallow, with a relatively sharp transition to a lower level of crosslinking. Alternatively, for example, the depth of surface crosslinking can be a significant fraction of the dimensions of the hydrogel-forming absorbent polymer, with a broader transition.

Depending on size, shape, porosity as well as functional considerations, the degree and gradient of surface crosslinking can vary within a given hydrogel-forming absorbent polymer. For particulate hydrogel-forming absorbent polymers, surface crosslinking can vary with particle size, porosity, etc. Depending on variations in surface:volume ratio within the hydrogel-forming absorbent polymer (e.g., between small and large particles), it is not unusual for the overall level of crosslinking to vary within the material (e.g., be greater for smaller particles).

Surface crosslinking is generally accomplished after the final boundaries of the hydrogel-forming absorbent polymer are essentially established (e.g., by grinding, extruding, foaming, etc.) However, it is also possible to effect surface crosslinking concurrent with the creation of final boundaries. Furthermore, some additional changes in boundaries can occur even after surface crosslinks are introduced.

A number of processes for introducing surface crosslinks are disclosed in the art. These include those where: (i) a di- or poly-functional reagent(s) (e.g., glycerol, 1,3-dioxolan-2-one, polyvalent metal ions, polyquaternary amines) capable of reacting with existing functional groups within the hydrogel-forming absorbent polymer is applied to the surface of the hydrogel-forming absorbent polymer; (ii) a di- or poly-functional reagent that is capable of reacting with other added reagents and possibly existing functional groups within the hydrogel-forming absorbent polymer such as to increase the level of crosslinking at the surface is applied to the surface (e.g., the addition of monomer plus crosslinker and the initiation of a second polymerization reaction); (iii) no additional polyfunctional reagents are added, but additional reaction(s) is induced amongst existing components within the hydrogel-forming absorbent polymer either during or after the primary polymerization process such as to generate a higher level of crosslinking at or near the surface (e.g., heating to induce the formation of anhydride and or esters crosslinks between existing polymer carboxylic acid and/or hydroxyl groups and suspension polymerization processes wherein the crosslinker is inherently present at higher levels near the surface); and (iv) other materials are added to the surface such as to induce a higher level of crosslinking or otherwise reduce the surface deformability of the resultant hydrogel. Combinations of these surface crosslinking processes either concurrently or in sequence can also be employed. In addition to crosslinking reagents, other components can be added to the surface to aid/control the distribution of crosslinking (e.g., the spreading and penetration of the surface crosslinking reagents.)

Suitable general methods for carrying out surface crosslinking of hydrogel-forming absorbent polymers according to the present invention are disclosed in US patent 4,541,871 (Obayashi), issued September 17, 1985; published PCT application WO92/16565 (Stanley), published October 1, 1992, published PCT application WO90/08789 (Tai), published August 9, 1990; published PCT application WO93/05080 (Stanley), published March 18, 1993; US patent 4,824,901 (Alexander), issued April 25, 1989; US patent 4,789,861 (Johnson), issued January 17, 1989; US patent 4,587,308 (Makita), issued May 6, 1986; US patent 4,734,478 (Tsubakimoto), issued March 29, 1988; US patent 5,164,459 (Kimura et. al.), issued November 17, 1992; published German patent application 4,020,780 (Dahmen), published August 29, 1991; and published European patent application 509,708 (Gartner), published October 21, 1992; to all of which particular reference is made.

### Crosslinking Agent

A crosslinking agent is used to crosslink the polymer material of the precursor particles of the absorbent macrostructure. A suitable crosslinking agent can be a nonionic crosslinking agents described in US patent 5,102,597 (Roe et al), issued April 7, 1992. These nonionic crosslinking agents include polyhydric alcohols (e.g., glycerol), polyaziridine compounds (e.g., 2,2-bishydroxymethyl butanoltris[3-(1-aziridine) propionate]), haloepoxy compounds (e.g., epicholorhydrin), polyaldehyde compounds (e.g., glutaraldehyde), polyamine compounds (e.g., ethylene amine), and polyisocyanate compounds (e.g., 2,4-toluene diisocyanate), preferably glycerol.

Preferred crosslinking agents are those which primarily provide crosslinking at portions of the surface of the absorbent precursor particles. Such crosslinking agents preferably have relatively large molecular size, and are preferably cationic. As a result, it is believed, such a crosslinking agent is unable to penetrate inside the absorbent particles, and therefore can only react with polymer material at the surface thereof effectively. It is possible that some such larger crosslinking agent can penetrate into the particle when the particle is swelled via the swelling agent.

Another preferred crosslinking agent is one which reacts very rapidly with the anionic, typically carboxy functional groups of the polymer material of the absorbent particles, even at a room temperature range (e.g., at from about 13°C to about 33°C ). As a result, fairly modest levels (e.g., as low as about 1% by weight of the particles) of such crosslinking agent are required to provide effective surface crosslinking of the polymer material present in the absorbent precursor particles.

A preferred crosslinking agent of the present invention, however, is an adduct of epichlorohydrin with certain types of monomeric or polymeric amines. These amino-epichlorohydrin adducts react with the polymer material of the absorbent precursor particles, and in particular the anionic, typically carboxy, functional groups of these polymer materials to form a covalent, ester-type bond. In other words, the amino-epichlorohydrin adduct serves to crosslink the polymer material present in the absorbent precursor particles. (The portions of the absorbent particle containing polymer material that has been effectively crosslinked with the amino-epichlorohydrin adduct swell less in the presence of aqueous body fluids relative to the other uncrosslinked portions of the particle.) Such cationic amino-epichlorohydrin adduct, especially a polymeric resin version, is relatively large such that preferential surface crosslinking is achieved. Such adduct with its cationic functional groups (e.g., azetedinium groups) can react rapidly with the polymer material at the room temperature range of preferably from about 13°C to about 33°C, more preferably from about 18°C to about 28°C, most preferably about 23°C.

As used herein, "cationic amino-epichlorohydrin adduct" refers to the reaction product between epichlorohydrin and a monomeric or polymeric amine such that the resulting reaction product has at least two cationic functional groups. These adducts can be in the form of monomeric compounds (e.g., the reaction product of epichlorohydrin and ethylene diamine), or can be in polymeric form (e.g., the reaction product between epichlorohydrin, and polyamide-polyamines or polyethyleneimines). The polymeric versions of these cationic amino-epichlorohydrin adducts are typically referred to as "resins."

One type of amino compound which can be reacted with epichlorohydrin to form adducts useful in the present invention comprises monomeric di-, tri- and higher amines having primary or secondary amino groups in their structures. Examples of useful diamines of this type include bis-2-aminoethyl ether, N,N-dimethylethylenediamine, piperazine, and ethylenediamine. Examples of useful triamines of this type include N-aminoethyl piperazine, and dialkylene triamines such as diethylenetriamine, and dipropylenetriamine.

Such amine materials are reacted with epichlorohydrin to form the cationic amino-epichlorohydrin adducts useful as crosslinking agents herein. Preparation of these adducts, as well as a more complete description of the adducts themselves, can be found in US patent 4,310,593 (Gross), issued January 12, 1982, and in Ross et al, J. Organic Chemistry, Vol. 29, pp. 824-826 (1964). To both of these documents particular reference is made.

In addition to monomeric amines, polymeric amines such as polyethyleneimines can also be used as the amino compound. A particularly desirable amino compound which can be reacted with epichlorohydrin to form preferred cationic polymeric adduct resins useful herein comprise certain polyamide-polyamines derived from polyalkylene polyamines and saturated C3-C10 dibasic carboxylic acids. Epichlorohydrln/polyamide-polyamine adducts of this kind are water-soluble, thermosetting cationic polymers which are well known in the art as wet strength resins for paper products.

In the preparation of polyamide-polyamines used to form this preferred class of cationic polymeric resins, a dicarboxylic acid is first reacted with a polyalkylene-polyamine, preferably in aqueous solution, under conditions such as to produce a water-soluble, long chain polyamide containing the recurring groups -NH(CnH2nHN)x-CORCO- where n and x are each 2 or more and R is the C1 to C8 alkylene group of the dicarboxylic acid.

A variety of polyalkylene polyamines including polyethylene polyamines, polypropylene polyamines, polybutylene polyamines and so on can be employed to prepare the polyamide-polyamine, of which the polyethylene polyamines represent an economically preferred class. More specifically, preferred polyalkylene polyamines used to prepare the cationic polymeric resins herein are polyamines containing two primary amine groups and at least one secondary amine group in which the nitrogen atoms are linked together by groups of the formula -CnH2n- where n is a small integer greater than unity and the number of such groups in the molecule ranges from two up to about eight and preferably up to about four. The nitrogen atoms can be attached to adjacent carbon atoms in the group -CnH2n- or to carbon atoms further apart, but not to the same carbon atom. Also contemplated is the use of such polyamines as diethylenetriamine, triethylene tetramine, tetraethylenepentamine, dipropylenetriamine, and the like, which can be obtained in reasonably pure form. Of all the foregoing, the most preferred are the polyethylene polyamines containing from two to four ethylene groups, two primary amine groups, and from one to three secondary amine groups.

Also contemplated for use herein are polyamine precursor materials containing at least three amino groups with at least one of these groups being a tertiary amino group. Suitable polyamines of this type include methyl bis(3-aminopropyl)amine, methyl bis(2-aminoethyl)amine, N-(2-aminoethyl) piperazine, 4,7-dimethyltriethylenetetramine and the like.

The dicarboxylic acids which can be reacted with the foregoing polyamines to form the polyamide-polyamine precursors of the preferred cationic polymeric resins useful herein comprise the saturated aliphatic C3-C10 dicarboxylic acids. More preferred are those containing from 3 to 8 carbon atoms, such as malonic, succinic, glutaric, adipic, and so on, together with diglycolic acid. Of these, diglycolic acid and the saturated aliphatic dicarboxylic acids having from 4 to 6 carbon atoms in the molecule, namely, succinic, glutaric and adipic are most preferred. Blends of two or more of these dicarboxylic acids can also be used, as well as blends of one or more of these with higher saturated aliphatic dicarboxylic acids such as azelaic and sebacic, as long as the resulting long chain polyamide-polyamine is water-soluble or at least water-dispersible.

The polyamide-polyamine materials prepared from the foregoing polyamines and dicarboxylic acids are reacted with epichlorohydrin to form the cationic polymeric amino-epichlorohydrin resins preferred for use herein as the crosslinking agent. Preparation of such materials is describe in greater detail in US patent 2,926,116 (Keim), issued February 23, 1960, US patent 2,926,154 (Keim), issued February 23, 1960, and US patent 3,332,901 (Keim), issued July 25, 1967, all of which are incorporated by reference.

The cationic polyamide-polyamine-epichlorohydrin resins preferred for use herein as crosslinking agents are commercially marketed by Hercules Inc. under the trade name Kymene® . Especially useful are Kymene® 557H, Kymene® 557LX and Kymene® 557 Plus, which are the epichlorohydrin adducts of polyamidepolyamines which are the reaction products of diethylenetriamine and adipic acid. They are typically marketed in the form of aqueous solutions of the cationic resin material containing from about 10% to about 33% by weight of the resin active.

### Substrate layer

The substrate layer 7 can provide a variety of functions. It can serve as a distributing means for improving the distribution of applied liquids to be absorbed into the macrostructure layer. Preferably, the liquid distribution properties of the substrate layer are substantially greater than those of the absorbent macrostructure, such that the composite has improved liquid distribution properties relative to the absorbent macrostructure alone. In preferred embodiments, the substrate layer 7 comprises a plurality of capillary elements having a length, preferably arranged substantially in parallel, for improving the distribution of the liquid along the lengths thereof.

The substrate layer 7 can also serve as a supporting means for the absorbent macrostructure layer by supporting the interconnected absorbent particles in the absorbent macrostructure. As described above, the absorbent gelling particles are connected to at least one of the other adjacent particles through the higher level crosslinked surface portion. The substrate layer can support the bonds or interconnection of the absorbent gelling particles by resisting the forces of stress and strain in the absorbent composite during use which might otherwise cause interconnected particles of the absorbent macrostructure layer to break apart. The supporting means is preferably one which has excellent wet strength, and can impart improved wet strength and wet integrity to the absorbent macrostructure layer; that is, the substrate is effective as a supporting means after the absorbent composite, and the substrate itself, has become wet with liquid. Support for the bonded or interconnected gelling particles is needed especially in this situation, where the gelling particles begin to swell after absorbing liquid, thereby placing substantial strain on the interparticle connections. Such support is especially important when the absorbent composite is used in an absorbent article such as a diaper or catamenial product where external forces can also act upon the structure to cause interconnected particles of the absorbent macrostructure to break apart. Therefore, the substrate layer can serve to improve both the dry integrity and strength, and the wet integrity and strength, of the absorbent composite.

The support function provided by a substrate can also permit less dense macrostructure layers. The less dense absorbent macrostructures have a higher percent void volume and larger pore openings between adjacent interconnected gelling particles, thus reducing the potential for gel blocking and reducing the stresses in the macrostructure as the particles absorb fluid and expand.

The substrate layer can be selected from various materials known in the art such as cellulose fibers, nonwoven webs, tissue webs, foams, polyacrylate fibers, apertured polymeric webs, synthetic fibers, elastomers, and the like. Most such substrates can serve both as a distributing means and a supporting means for the absorbent macrostructure layer. Preferably, the substrate layer is comprised of cellulosic material or a material having cellulosic functionality. Preferred substrates for use as a fluid distributing means can be selected from cellulosic materials, fibrous webs, cellulosic fibrous webs, solid foams, cellulosic foams, and polyvinyl alcohol foams. Preferred substrates for use as a supporting means can be selected from cellulosic materials, fibrous webs, nonwoven webs, fabrics, cellulosic fibrous webs, solid foams, cellulosic foams, and polyvinyl alcohol foams.

The substrate layer 7 is preferably flexible and pliable to encourage such properties in the resulting absorbent composite. A substrate layer can be substantially resilient and non-stretchable, or it can be stretchable or deformable to a varying extent in response to forces exerted normal to and in the plane of the surface of the substrate.

The thickness and basis weight (weight per unit area of substrate) of a substrate material will vary depending on the type of substrate and the desired properties. A substrate can also comprises a plurality of individual sheets, or plies, of a particular substrate material, or a combination of one or more substrate layers in a laminate. As a typical substrate, a Bounty® sheet has a thickness of from about 0.02 mm to about 1.2 mm, more preferably from about 0.3 mm to about 0.8 mm, and a basis weight of from about 5 gm/m² to about 100 gm/m², more preferably from about 10 gm/m² to about 60 gm/m², and most preferably from about 15 gm/m² to about 40 gm/m². As another typical substrate, a cellulose foam has a dry compressed thickness of from about 0.5 mm to about 3.0 mm, more preferably from about 0.8 mm to about 2.0 mm, a wet expanded thickness of from about 0.8 mm to about 6.0 mm, more preferably from about 1.0 mm to about 5.0 mm, and a basis weight of from about 50 gm/m² to about 2,000 gm/m², more preferably from about 100 gm/m² to about 1,000 gm/m².

Substrates for use as support means typically have a dry tensile strength of from about 500 gm/in to about 8,000 gm/in, though more preferably from about 1,000 gm/in to about 3,000 gm/in, a wet tensile strength of from about 200 gm/in to about 5,000 gm/in, though more preferably from about 400 gm/in to about 1,000 gm/in, and a wet burst strength of from about 100gm to about 2,000 gm, though more preferably from about 200 gm to about 1,000 gm,

In preferred embodiments, the substrate layer comprises a cellulosic fibrous web such as paper towelling and paper tissue.

Examples of such cellulosic fibrous webs are disclosed in US patent 3,953,638, issued April 27, 1976, US patent 4,469,735, issued Sept. 4, 1984, US patent 4,468,428, issued Aug. 28, 1984, and US patent 4,986,882, issued Jan. 22, 1991, to all of which particular reference is made. A preferred example of such is Bounty® paper towel, commercially marketed in the U.S. by The Procter & Gamble Company. Another preferred example of such is Kinocloth® paper tissue, commercially marketed in the U.S. and Japan by Honshu Paper Co., Ltd.. Bounty® and Kinocloth® are hydrophilic and have good distribution and wicking properties, as well as good wet integrity.

In another preferred embodiment, the substrate layer comprises a cellulosic foam. In general, a cellulosic foam will provide a higher liquid wicking rate over a longer wicking distance than a cellulosic fibrous web. Preferably, the cellulosic foam is in a compressed state so as to further improve its wicking and fluid distribution properties. Suitable cellulose foam can be made of regenerated rayon fibers by well-known methods, such as those disclosed in European patent Publication (Publication No. 0,293,208). Such cellulose foams have numerous small cells, the size of which affect the capillarity and absorptivity of the foam. The cellulose foam layer will ordinarily, and preferably, expand when wet. A preferred cellulosic foam is one which has been compressed in the dry state prior to use. The average pore size of the cellulose foam layer can be determined by the compression. In preferred embodiments, the average pore size of the cellulose foam layer, as measured in the dry state after any compression, is from about 1 micron to about 1000 microns, preferably from about 1 micron to about 200 microns, more preferably from about 5 microns to about 70 microns. A preferred compressed cellulose foam layer has a density of from about 0.1 g/cc (about 0.05 g/in³) to about 0.8 g/cc (about 0.41 g/in³) and has a compressed thickness (in sheet form) of from about 2 mm to about 5 mm. In general, better wicking properties can be obtained by using a foam layer having a higher density, or a smaller pore size. When such compressed cellulose foam layer contacts with liquids, the pore size of the foam begins to expand whereby the thickness of the foam layer become increased.

Absorbent foam substrates, particularly compressed cellulose foam substrates, are highly preferred substrates in the absorbent composites of the present invention. In addition to having excellent dry and wet strength and integrity, cellulose foam substrates, specifically in the form of sheets, have excellent capillarity and fluid wicking properties. When liquid such as water or body exudate is deposited onto the surface of an absorbent composite comprising a foam cellulose substrate the liquid passing through the absorbent macrostructure layer and into the cellulose foam substrate is distributed quickly outward toward dry foam areas in cellulose foam layer due to its capillary suction. That is, as the cellulose foam absorbs water or aqueous liquids, the cellulose foam cell structure begins to expand. Since the dry foam areas have a cell structures which are still compressed and which are smaller than the cells of the wetted areas, fluids readily wick into the dry foam areas. Such cellulose foam substrates are characterized by excellent fluid wicking and distribution. Specifically, such cellulose foam substrates have fast wicking ratio or speed (for example, up to at least 12 cm wicking distance in 4 minutes in a vertical wicking test) and long wicking distance capability (for example, from about 20 cm to about 30 cm in the first one hour in a vertical wicking test).

In yet another embodiment, the substrate layer can be a cellulose foam substrate formed by depositing cellulose foam particles ( or granules ). The cellulose foam particles have an average volume of at least about 0.1 mm³, preferably from about 1.0 mm³ to about 125 mm³. Preferably, the cellulose foam particles are deposited and compacted on an absorbent macrostructure layer.

A cellulose foam substrate is particular preferred when using the absorbent composite of the present invention in an absorbent catamenial article. When blood is deposited onto a cellulose foam substrate layer of an absorbent composite, the cellulose foam substrate can serve to acquire the blood, filter aggregates from the blood, and distribute the remaining liquid portion of the blood to the absorbent macrostructure layer below. The cellulosic foam substrate may be slitted in various patterns as for instance described in EP-A-0 293 208 to increase the substrate's flexibility.

In yet another preferred embodiment, the substrate layer comprises a compressed or non-compressed polyvinyl alcohol foam. In general, such foam preferably has properties and structure substantially as the cellulosic foam above.

### Examples

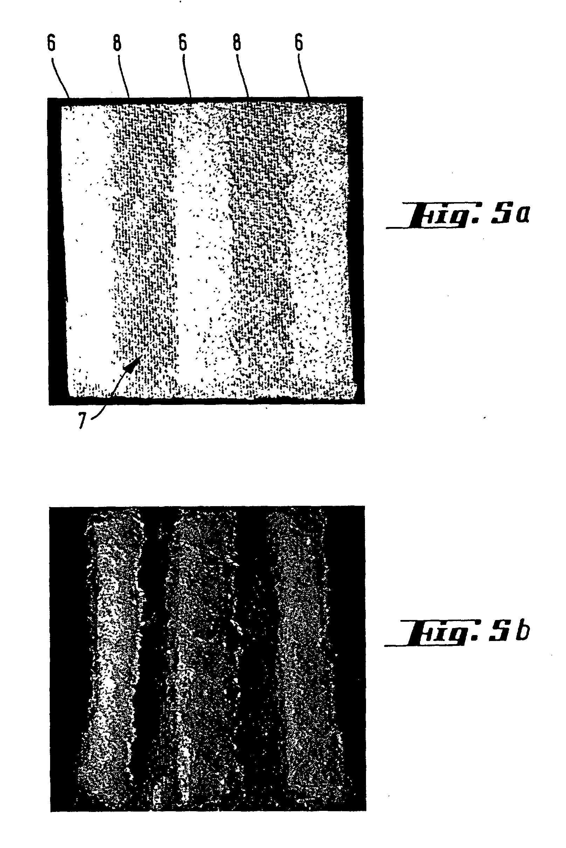

Figures 5a-5c show reflection-mode photographs of an absorbent structure according to the invention during different stages of swelling. The structure of figure 5a was produced by depositing 3 g of absorbent polymeric particles produced by Nippon Shokubai under tradename Acqualic CA L761f onto a BOUNTY® substrate tissue 7 of 10 cm x 10 cm as produced by The Procter & Gamble Company. Then a crosslinking solution comprising 10 % by weight of Kymene® 557 Plus, 40 % by weight of glycerol and 50 % by weight of water, was applied in a striped pattern in the amount of 0.22 g of solution per g of absorbent polymeric particles. The crosslinking agent was reacted with the polymeric particles for about 24 hours at room temperature. In the regions 6 where the crosslinking agent was applied, the polymeric particles were mutually connected by interparticle crosslink bonds and were connected to the tissue 7 by the crosslinking agent. The particles in the regions 8, where no crosslinking agent was applied, remained unattached and were removed from the tissue 7. Figure 5b shows the swelling of the crosslinked polymereic particles after wetting the sample of figure 5a with 30 ml of 0.9 % saline solution after 1 minute. Clearly it can be seen that channels are formed in regions 8. Figure 5c shows the sample of figure 5a after 5 minutes from application of the saline solution. From figure 5c it appears that also in the fully swollen state, wherein the absorbent capacity of the polymeric particles in this case is about 17g/g, the channels are still maintained: Thereby liquids can be transported across the absorbent structures via the channels to those regions where the polymeric particles have not yet reacted their full capacity and liquids can still be absorbed.

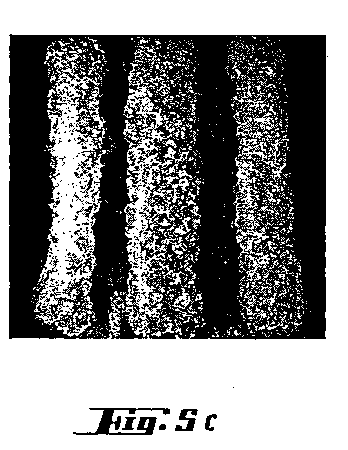

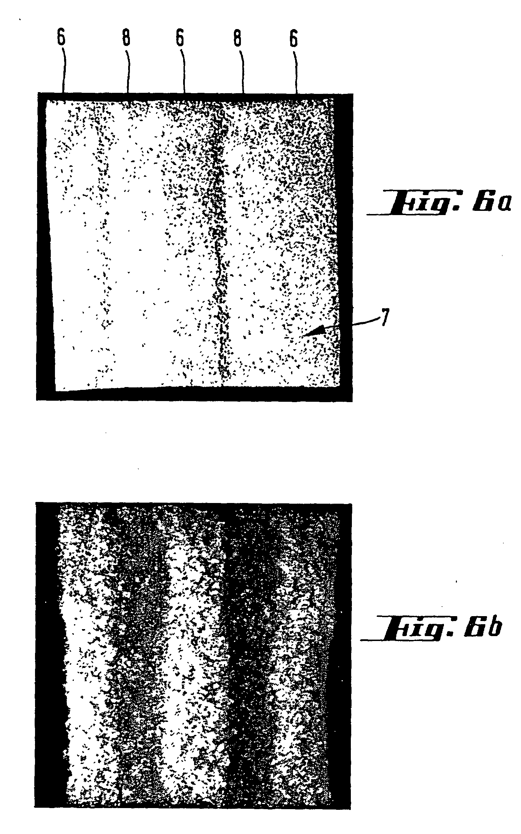

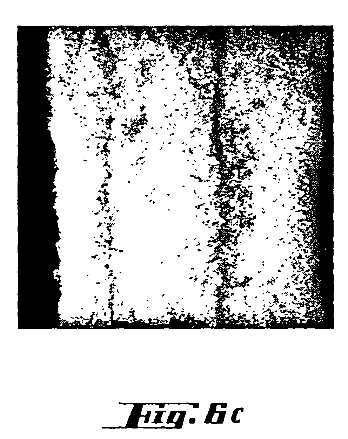

Figures 6a-6c show reflection-mode photographs of an absorbent structure according to the invention, wherein 3 g of polymeric particles of type were deposited onto a 10 cm x 10 cm tissue of the same type as described above. In the regions 8 a crosslinking solution was applied comprising weight percentages of: 18 % Kymene® 557 Plus, 40 % glycerol and 32% water in the amount of 0.22 g of crosslinking agent per g of absorbent polymeric particles. In the regions 8 the polymeric particles are mutually connected to form an interpartically crosslinked aggregate. The application of the crosslinking agent to the particles in the regions 8 results in an increased degree of crosslinking. In the regions 6 a solution comprising 40 % by weight glycerol and 60 % by weight water was applied, no Kymene® 557 Plus solution being present. The polymeric particles in the regions 6 are attached to the tissue 7, but are not mutually attached by inter-particle crosslink bonds. The polymeric particles in the regions 6 are mutually interconnected by hydrogen bonds, which dissolve upon wetting. The particles in the regions 6 have a relatively low degree of interparticle crosslinking and surface crosslinking compared to the particles in regions 8. The particles in the regions 8 are bonded to the tissue by the glycerol-water solution and remain attached to the tissue, so that the absorbent structure of figure 6a has a substantially uniform basis weight of polymeric particles. Figure 6b shows formation of channels 1 minute after wetting of the structure of figure 6a with 30 ml of an 0.9 % saline solution, the dark areas in figure 6b being the lower parts. The particles in the regions 8 will due to the relatively high degree of crosslinking compared to the particles in the regions 6, swell less rapidly than the particles in the regions 6. Hence channels are formed through which liquids can be transported. The channels also form void space 8 into which the rapidly swelling material from the regions 6 can expand, so that bending and curling of the absorbent structure is prevented. Figure 6c shows the absorbent structure of figure 6a in its fully swollen state 5 minutes after depositing the saline solution onto the structure. It can be seen that the channels in the region 8 are filled with gelled polymeric particles both from regions 8 and from regions 6.

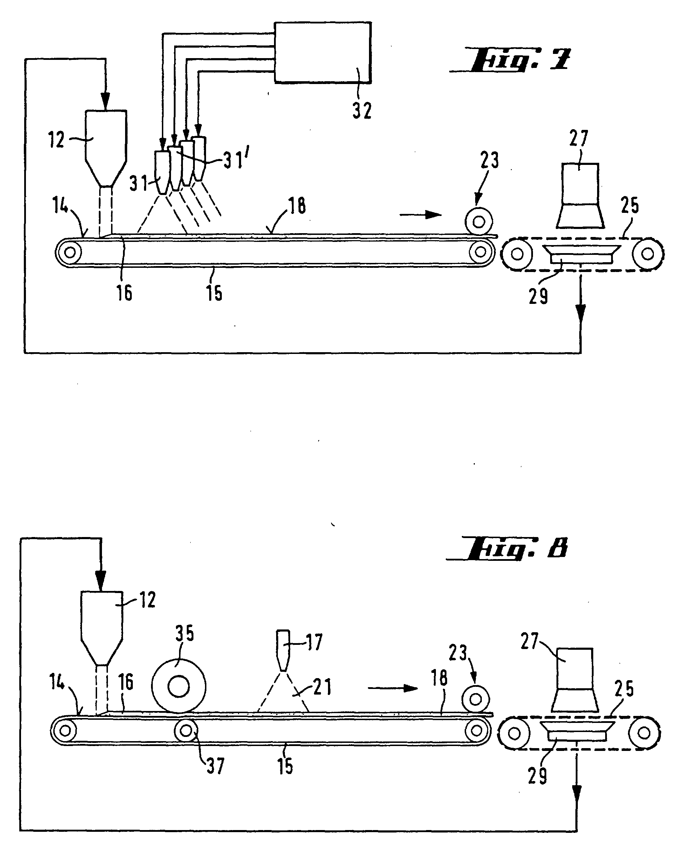

Figure 7 shows a an alternative way of applying different amounts of crosslinking agent by using a multiplicity of spray nozzles 31, 31'. The nozzles 31,31' are arranged across the width of the conveyor belt 15 in a direction perpendicular to the plane of the drawing. A control unit 32 sets the rate of emission of each spray nozzle 31,31' in such a manner that a pattern of crosslinking agent is applied which extends perpendicular to the plane of the drawing. By periodically varying the emission rate of each nozzle 31,31' in time, the amount of crosslinking agent that is applied to the deposition surface is varied in the direction of transport of the conveyor belt 15. In this way patterns of crosslinking agent are applied which vary both in the direction of the width of the conveyor belt 15 and in the direction of transport of the conveyor belt.

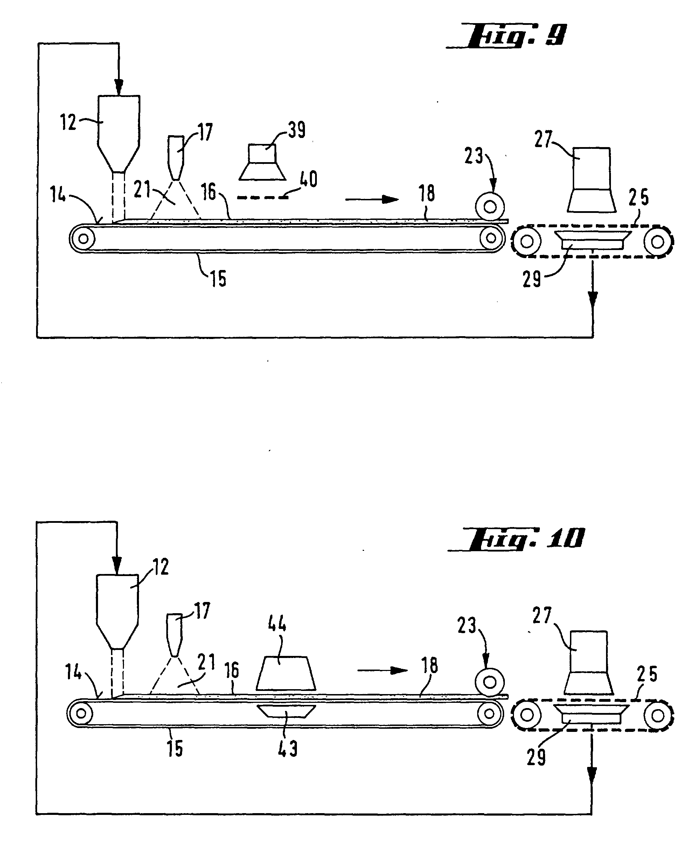

Figure 8 shows an embodiment of a process in which the layer of particles 16 is compacted in certain areas prior to crosslinking of the particles, in a nip between rolls 35 and 37. The roll 35 is provided with a pattern of peripheral projections and recesses, such as a number of circumferential grooves. By compacting the particles in predetermined areas, the number of crosslink bond in the compacted zones can be increased. In the non-compacted areas, less crosslink bonds are formed.

Figure 9 shows an embodiment in which the crosslinking step is initiated by the application of ionizing radiation from a source 39. After uniform application of the crosslinking agent from spray nozzle 17, the source 39 irradiates the deposition surface 14 with a pattern of radiation such that the energy that is deposited per unit area varies across the deposition surface 14. The use of ionising radiation in polymerisation processes has been described in detail in EP-A-054 841 and in US patent US-A-4,646,730.

In case the source 39 is formed by a light source, an x-ray tube or a source of gamma radiation, such as a Cobalt source, a mask 40 is projected onto the deposition surface 14. The mask 40 has transparent and opaque areas . The rate of crosslinking will be largest at the projection of the transparent areas onto the deposition surface.

In case a laser beam or electron beam is used to irradiate the deposition surface 14, the electron beam or laser beam can be scanned across the deposition surface in a desired pattern. The mask 40 can in this case be omitted.

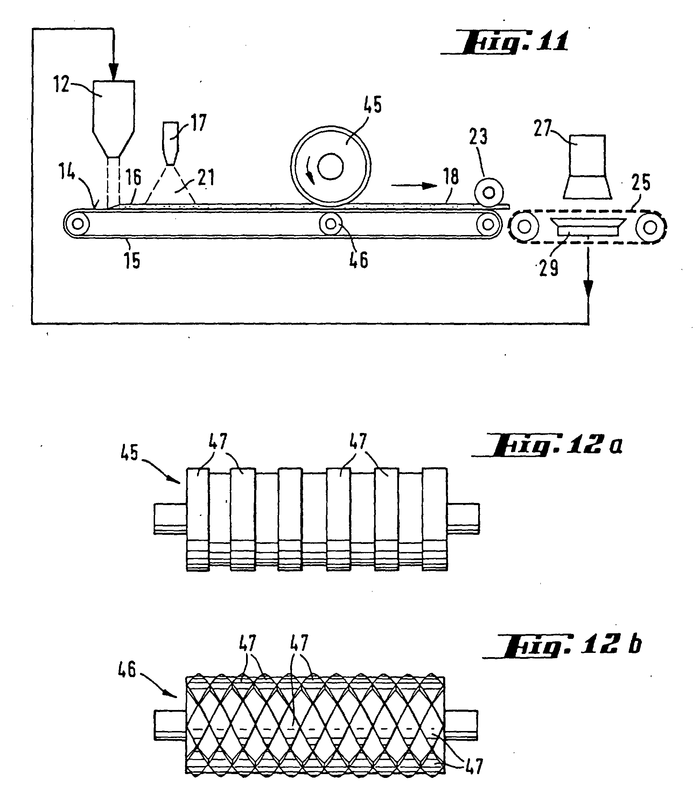

Figure 10 shows an embodiment of a process wherein after uniform application of crosslinking agent across the deposition surface 14, the layer of particles 16 is passed under a heat curing unit 44,43. The heat curing unit can comprise an array of heated elements, in the form of a pattern of studs or a grid. The conveyor belt is intermittently slowed down or stopped at the location of the curing unit 44,43, which is subsequently activated. An apparatus for periodically slowing down or stopping only a specific part of the conveyor belt, while continuously moving the belt, has been described in PCT publication WO 95/12539. The upper element 44 may be moved downward and contact the layer of particles 16 or may be located in close proximity to the layer of particles 16.

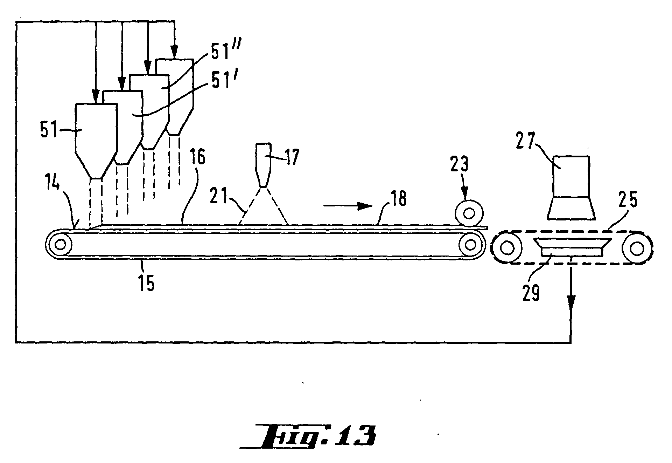

Figure 11 shows a curing unit which comprises two rolls 45, 46. After application of the crossslinking agent, the layer of particles 16 is passed between the nip formed by the rolls 45, 46. The upper roll 45 comprises a peripheral pattern of raised and recessed areas as shown in figures 12a and 12b, which show axial side elevational views of the roll 45. In figures 12a and 12b, the recessed areas are indicated by dark shading. Due to the higher pressure on the particles that are located below the projections 47 on the roll 45, the layer 16 is in these areas compacted. In the compacted areas the number of crosslink bonds that are formed is higher than the number of crosslink bonds formed at the areas of the deposition surface 14 which are located underneath the recesses of the roll 45. Alternatively, the roll 45 may be heated to further increase the number of crosslink bonds formed underneath the projections 47, through which heat transfer to the layer of particles 16 is largest.

Figure 13 shows an embodiment of a process wherein an array of applicators 51,51',51" is located across the deposition surface 14, the array extending perpendicular to the plane of the drawing. The applicators 51, 51', 51" deposit varying basis weights of particles onto the deposition surface. Alternatively, the applicators 51,51',51" deposit particles of different particle size or particles of different chemical composition onto the deposition surface 14. After laydown, homogeneous application of crosslinking agent by spray nozzle 17, results in a non-homogeneous distribution of the degree crosslinking across the deposition surface 14.

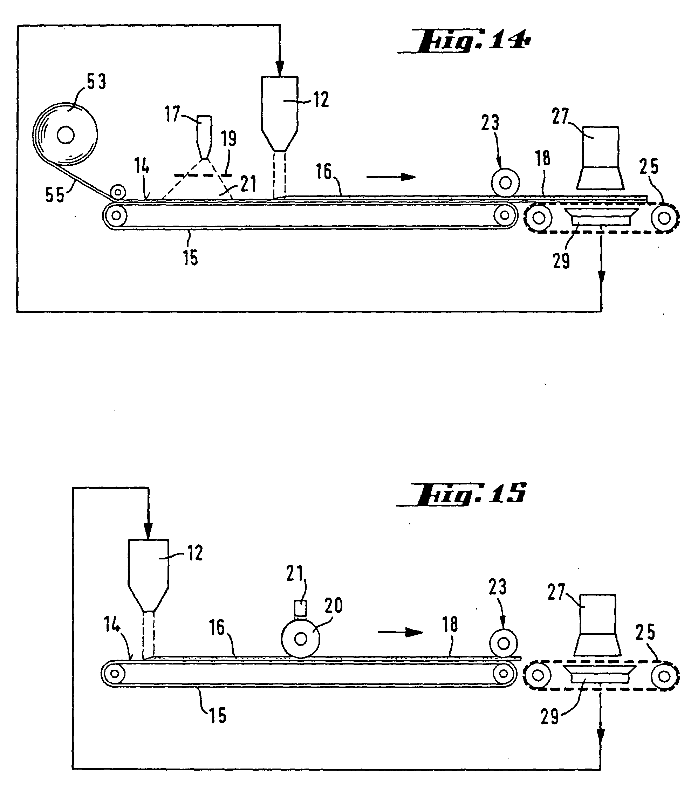

Figure 14 shows a process for making an absorbent structure according to the invention wherein the deposition surface 14 is formed by a tissue 55 which is unwound from a roll 53. In this embodiment, the tissue is sprayed by a pattern of crosslinking agent by the spray nozzle 17, which is located upstream of the applicator 12. As a consequence, the number of inter particle crosslink bonds between the particles 5 is largest in the areas of the tissue 55 that have been wetted with the crosslinking agent. The basis weight of the particles may be low enough such that no interparticle crosslink bands are formed, as described in PCT publication WO 96/07476.

Figure 15 shows an apparatus comprising an alternative applicator means 20,21 for providing a pattern of crosslinking agent to the deposition surface 14, which is similar to a "gravure printing" step. The applicator means comprise a rotating roll 20 which rotates along a supply unit 21 from which the periphery of the roll 20 is wetted with the crosslinking agent. The periphery of the roll 20 is provided with cells or grooves in which the crosslinking agent is rotated towards the layer of particles 16, and from which the crosslinking agent is deposited.

In the methods as described in figures 7 to 11 and 13 to 15, the conveyor belt 15 can be driven in continuous motion or can be periodically stopped or slowed down. The application of crosslinking agent can in the latter case be synchronised with the motion of the conveyor belt 15. The mask 19 may also be formed as a rotating belt which is rotated with the same speed as the conveyor belt 15, as shown in the enlarged inset of figure 4.

Figure 16 shows an embodiment of an absorbent structure in which the polymeric particles 5 have been removed from the regions 13,13' of lower degrees of crosslinking. The regions 13,13' completely separate the regions 11,11' of higher crosslinking. In case the regions 13, 13' which do not comprise any polymeric particles, extend along the whole length (the direction perpendicular to the plane of the drawing), the presence of the substrate 7 is essential to maintain a unitary, self supporting structure.

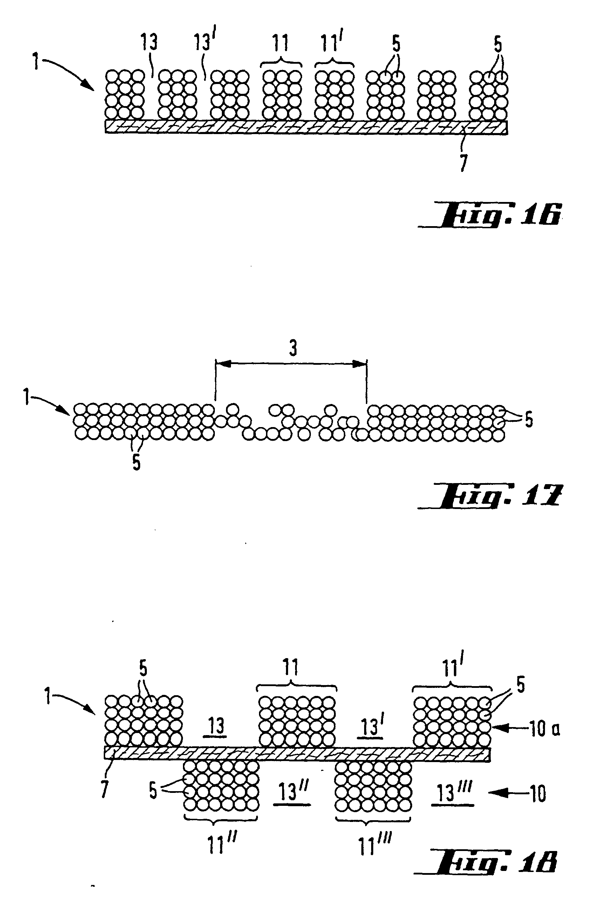

Figure 17 shows a structure in which in the central region 12, the density of the particles 5 is relatively low. In this manner a central window for rapid uptake of liquids is provided.

In the embodiment of figure 18, both sides of the substrate 7 are covered by interparticle crosslinked macrostructures 10, 10a which comprise regions 11-11''' of a high degree of interparticle crosslink bonds, which regions are separated by regions 13-13"' without polymeric particles 5. On forming the structure of figure 18, first the lowest layer 10 of particles may be deposited onto the conveyor belt 15. Subsequent crosslinking agent is applied to this lower layer in the desired pattern, after which curing is effected and the particles which comprise a relatively low number of interparticle crosslink bonds are removed by the air gun 27 and suction box 29. After that, the lower layer 10 is covered with the substrate 7. The substrate may be provided with a layer of adhesive, to attach to lower layer 10 of interpartically crosslinked particles or may be attached by crosslink bonds that are formed between the layer 7 and the layer 10. Then, the particles in the upper layer 8 are laid down onto the substrate 7 in the manner according to the embodiments of any of the figures 7 to 11, 13 or 15.

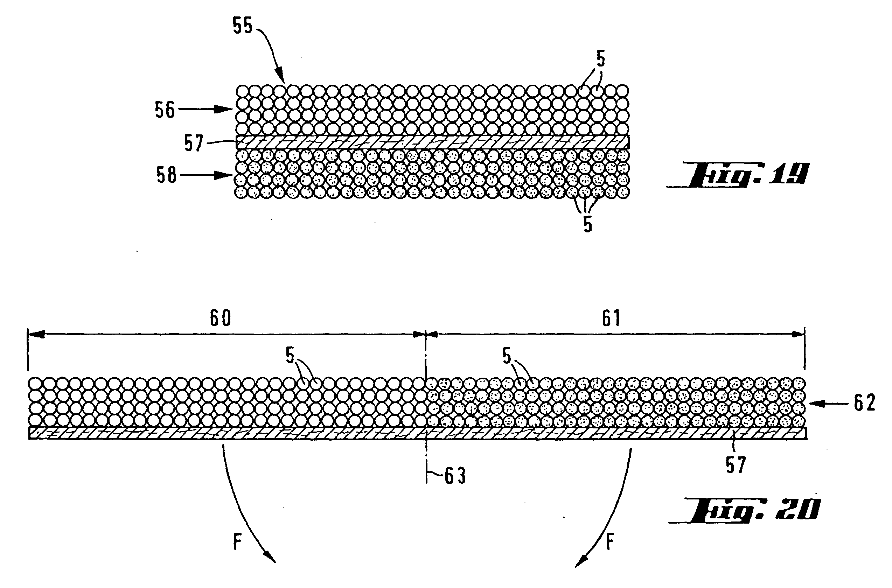

Figure 19 shows an embodiment of a multilayer structure comprising two layers 56, 58 of interparticle crosslinked aggregates, the lower layer 58 having a lower degree of crosslinking than the upper layer 56. The absorbent structure according to figure 19 can be made in a manner similar to the one by which the structure of figure 18 is formed.

Alternatively, the structure of figure 19 can be formed by folding the structure as shown in figure 20 in the direction of the arrows F, around a folding line which is located perpendicular to center line 63 and perpendicular to the plane of the drawing. The interparticle crosslinked macrostructure 62 is formed by the method as described in any of figures 4, 7 to 11 or 13 to 15. The number of interparticle crosslink bonds in the region 60 is higher than the number of interparticle crosslink bonds in the region 61.

In the preceding examples preferred absorbent structures have been described comprising interpartically crosslinked macrostructures. The invention is however not limited to interpartically crosslinked macrostructures. The method as shown in figure 4, figures 7 to 11 and figures 13 to 15 can be easily modified to form absorbent structures comprising polymeric particles which are deposited onto a substrate 7 without interparticle crosslink bonds being formed. The particles may for instance be adhesively connected to the substrate 7, e.g. by placing a glue applicator upstream of the particle applicator 12, the glue applicator providing a liquid permeable layer of adhesive to substrate 7. By effecting different degrees of surface crosslinking of the particles which are adhesively attached to a substrate 7, the advantageous liquid-handling effects caused by different absorption properties of the particles across the surface of the absorbent structure can be obtained.

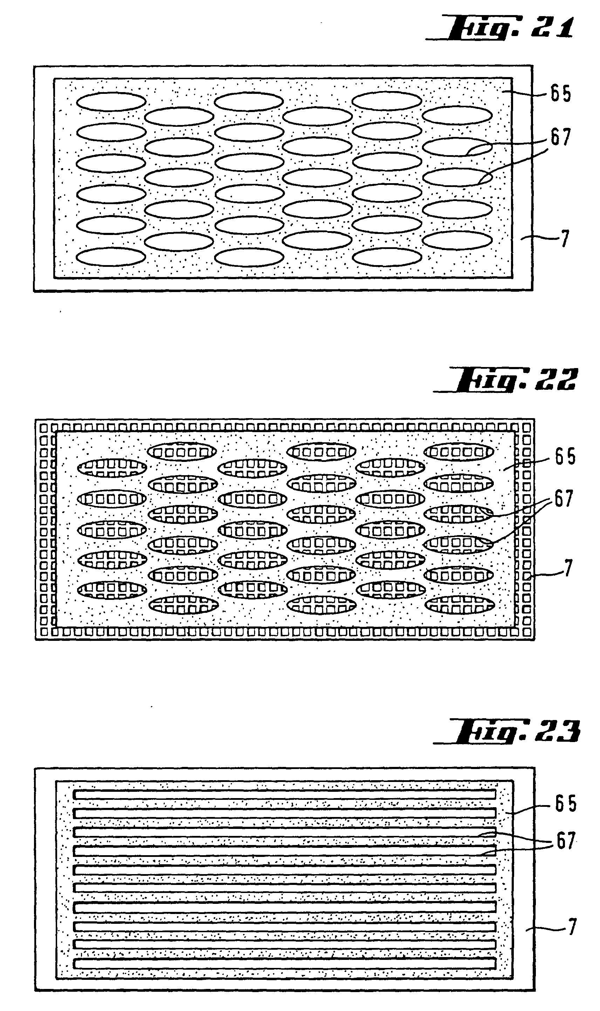

The absorbent structures of figures 21 and 22 comprise a number of holes 67 distributed across the interparticle crosslinked macrostructure 65. Through the holes 67, liquid can rapidly enter into the substrate layer 7 and can be transported in the substrate layer. As shown in figure 22, the substrate layer 7 may itself be of a relatively open structure. Absorbent structures as described in figures 21 and 22 may advantageously be used as upper layers in a multilayer absorbent product such as described for instance in WO 92/11831 (Feist) or US patent no. 5,304,161 (Noel et al.). In the multi-layer absorbent products as described in the above references a lower storage layer comprising absorbent polymeric particles is located below an upper layer of absorbent polymeric particles. The upper layer comprises passage ways for liquids to enter into the absorbent product and to flow into the lower storage layer, and may be formed by the structures of figures 21 to 26.

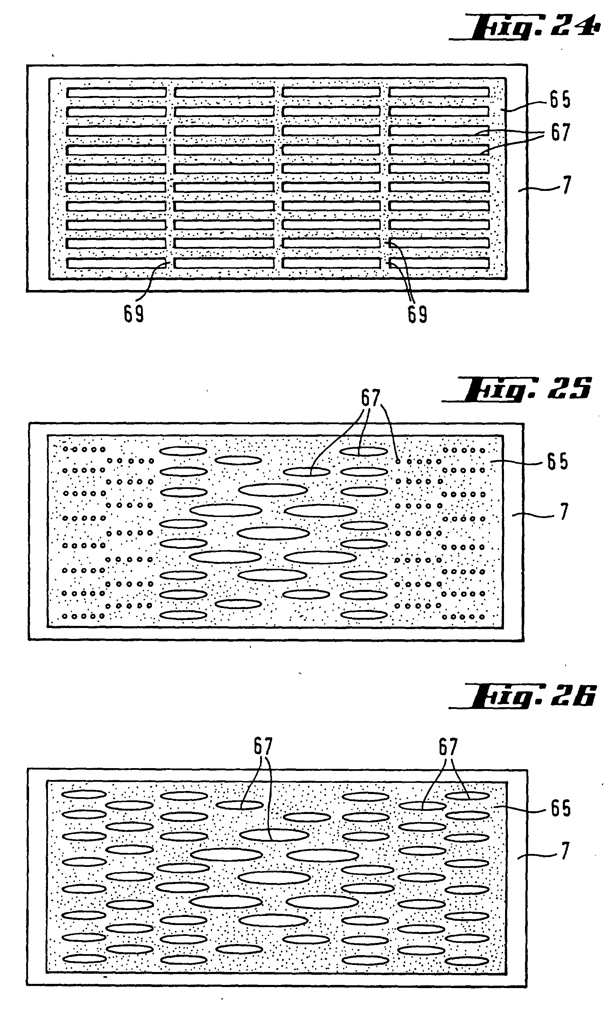

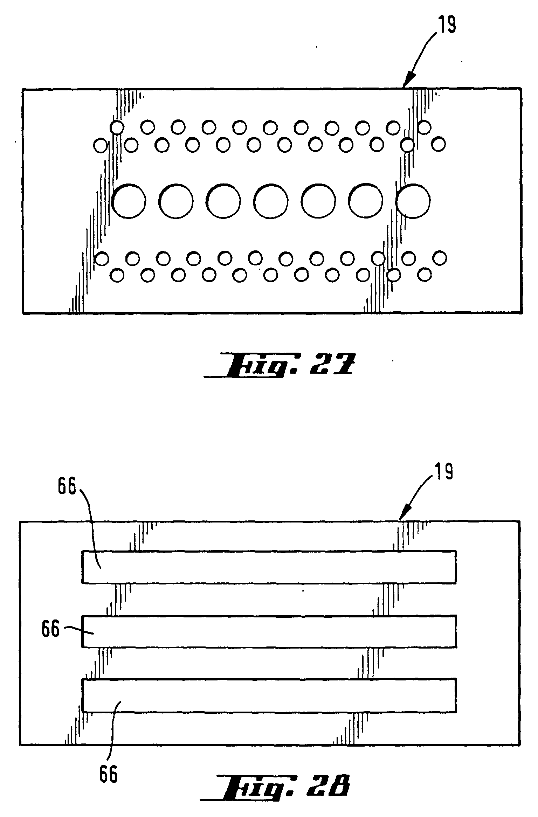

In the absorbent structures of figures 23 and 24 longitudinal channels 67 are formed in the interparticle crosslinked macrostructure 65 to promote liquid flow along the channels. In the structure of figure 24, reinforcing elements 69 run across the channels 67, such that the structural integrity is improved. The structures of figures 23 and 24 may for instance be used as the absorbent core of a thin sanitary napkin as described in US Patent No. 4,950,264. In the absorbent structures of figures 25 and 26, the size of the holes 67 varies across the surface of the crosslinked macrostructure 65. In absorbent products these structures can be used to provide an absorbent core which can rapidly absorb liquid into the core via the larger holes in the specific loading area of the product. By providing holes 65 of the required size in specific locations, absorbent products can be tailored to best suit a specific category of users such as infants or adults, or male or female users.

Figures 27 and 28 show a plan view of masks 19 that can be located between the spray nozzle 17 and the deposition surface 14. In the mask 19 according to figure 28, a number of slits 66 is present. The slits 66 extend parallel to the length of the conveyor belt 15 when placed between the nozzle 17 and the deposition surface 14. As shown if figure 27, the mask 19 may comprise a number of holes, the size and/or the pitch of which varies across the mask 19. If the deposition surface 14 is moved underneath the spray nozzle 17 and the masks19 of figure 27 or figure 28 in a continuous manner, an absorbent structure having a striped distribution of absorbent polymeric particles as shown in figure 23 is formed. If the conveyor-belt 15 is periodically stopped when the crosslinking agent is applied through the mask as shown in figure 27, a pattern of the type as shown in figure 25 or 26 may be formed. Instead of periodically stopping the conveyor belt 15, the mask 19 may be translated or rotated parallel to the conveyor belt such that the relative velocity between the mask 19 and the conveyor belt is zero.

## Claims

1. Method of making an absorbent macro-structure having a circumscribed volume of not smaller than 10 mm³, said method comprising the steps of
a) providing at least one plurality of absorbent polymer particles (APP);
b) providing a carrier;
c) disposing said particles on said carrier to form a macro-structure comprising a first and second region;
d) providing one or more cross-linking agent(s) suitable to cross-link the absorbent polymer particles (APP) on their surface and/or across particles;
e) applying said cross-linking agent(s) to said APP in at least one of said regions;
f) adjusting the reaction conditions to enable cross-linking reactions; **characterized in that** one or more of said steps are executed differently in said first and said second region so as to create **regions having distinct liquid handling properties by creating** different degree of surface cross-linking and/or interparticle cross-linking on or between said absorbent polymer particles (APP) in said first and second region.

2. Method according to claim 1 or 2, wherein the absorbent polymer particles (APP) of step a) are different in said first and the second regions for one or more of the following:
a1) chemical composition of the absorbent polymer particles (APP);
a2) physical shape, average particle size, and/or average particle size distribution.
a3) density or basis weight

3. Method according to any of the preceding claims, wherein the density of the absorbent polymer particles (APP) in the first region is between 0.5 and 2 times the density of the absorbent polymer particles (APP) in the second region.

4. Method according to any of the preceding claims, wherein said cross-linking agents as provided in step d) in said first and second regions differ in one or more of the following:
d1) chemical composition of the agent;
d2) concentration of the agent.

5. Method according to any of the preceding claims, wherein said application of said cross-linking agents in step e) is different for said first and said second region in one or more of the following:
e1) amount of applied crosslinking agent
e2) type of application

6. Method according to claim 5, wherein said amount of applied cross-linker per gram of absorbent polymer particles (APP) in one regions is at least twice the amount of the other region.

7. Method according to any of the preceding claims, wherein said adjustment of conditions for cross-linking reaction as for step f) is different for said first and second region in one or more of the following:
f1) energy input to the reactants;
f2) duration of reaction conditions
f3) pressure under which reaction occurs.

8. Method according to claim 7, wherein said input is in form of electro-magnetic irradiation, emitted electrons, or heat derived from a curing unit.

9. Method according to claim 7 or 8, wherein said energy input deposited in said regions is different.

10. Method according to claim 9, wherein the difference in energy input is achieved by use of a mask is placed between the absorbent polymer particles (APP) and said curing unit with different masking areas of varying opacity for the electro-magnetic irradiation, emitted electrons, or heat.

## Patentansprüche

1. Verfahren zum Herstellen einer absorbierenden Makrostruktur mit einem umschriebenen Volumen von nicht kleiner als 10 mm³, wobei das Verfahren die Schritte umfaßt
a) Bereitstellen wenigstens einer Mehrzahl von absorbierenden Polymerteilchen (APP);
b) Bereitstellen eines Trägers;
c) Anordnen der Teilchen auf dem Träger, um eine Makrostruktur mit einem ersten und einem zweiten Bereich zu bilden;
d) Bereitstellen ein oder mehrerer vernetzender Mittel, die dazu geeignet sind, die absorbierenden Polymerteilchen (APP) auf ihrer Oberfläche und/oder quer zu den Teilchen zu vernetzen;
e) Aufbringen des/der vernetzenden Mittel(s) auf das APP in wenigstens einer der Bereiche;
f) Einstellen der Reaktionsbedingungen, um Vernetzungsreaktionen zu ermöglichen;
**dadurch gekennzeichnet, daß** ein oder mehrere der Schritte in dem ersten und dem zweiten Bereich unterschiedlich ausgeführt werden, um so Bereiche mit unterschiedlichen Flüssigkeits-Handhabungseigenschaften zu erzeugen, indem ein unterschiedlicher Grad der Oberflächenvernetzung und/oder interpartikulären Vernetzung auf oder zwischen den absorbierenden Polymerteilchen (APP) in dem ersten und dem zweiten Bereich erzeugt wird.

2. Verfahren nach Anspruch 1 oder 2, in welchem die absorbierenden Polymerteilchen (APP) in Schritt a) in dem ersten und dem zweiten Bereich in ein oder mehreren der folgenden Punkte unterschiedlich sind:
a1) chemische Zusammensetzung der absorbierenden Polymerteilchen (APP);
a2) physische Form, mittlere Teilchengröße und/oder mittlere Teilchengrößenverteilung;
a3) Dichte oder Basisgewicht.

3. Verfahren nach einem der vorstehenden Ansprüche, in welchem die Dichte der absorbierenden Polymerteilchen (APP) im ersten Bereich zwischen dem 0,5 und 2-fachen der Dichte der absorbierenden Polymerteilchen (APP) im zweiten Bereich beträgt.

4. Verfahren nach einem der vorstehenden Ansprüche, in welchem sich die Vernetzungsmittel, wie sie im Schritt d) bereit gestellt werden, im ersten und zweiten Bereich in ein oder mehreren der folgenden Punkte unterscheiden:
d1) chemische Zusammensetzung des Mittels;
d2) Konzentration des Mittels.

5. Verfahren nach einem der vorstehenden Ansprüche, in welchem die Anwendung der Vernetzungsmittel im Schritt e) für den ersten und den zweiten Bereich in ein oder mehreren der folgenden Punkte unterschiedlich ist:
e1) Menge des aufgebrachten Vernetzungsmittels;
e2) Art der Aufbringung.

6. Verfahren nach Anspruch 5, in welchem die Menge des aufgebrachten Vernetzers pro Gramm der absorbierenden Polymerteilchen (APP) in einem Bereich wenigstens das Doppelte der Menge des anderen Bereichs beträgt.

7. Verfahren nach einem der vorstehenden Ansprüche, in welchem die Einstellung der Bedingungen für die Vernetzungsreaktion gemäß Schritt f) für den ersten und den zweiten Bereich in ein oder mehreren der folgenden Punkte unterschiedlich ist:
f1) Energieeintrag zu den Reaktanden;
f2) Dauer der Reaktionsbedingungen;
f3) Druck, unter welchem die Reaktion auftritt.

8. Verfahren nach Anspruch 7, in welchem der Eintrag in Form einer elektromagnetischen Bestrahlung, emittierter Elektronen oder aus einer Aushärtungseinheit abgeleiteter Wärme erfolgt.

9. Verfahren nach Anspruch 7 oder 8, in welchem der in den Bereichen abgesetzte Energieeintrag unterschiedlich ist.

10. Verfahren nach Anspruch 9, in welchem die Differenz des Energieeintrags durch Verwendung einer Maske erreicht wird, welche zwischen den absorbierenden Polymerteilchen (APP) und der Aushärtungseinheit mit unterschiedlichen Maskierungsflächen variierender Opazität für die elektromagnetische Bestrahlung, emittierten Elektronen oder Wärme angeordnet ist.

## Revendications

1. Procédé pour préparer une macrostructure absorbante ayant un volume circonscrit non inférieur à 10 mm³, ledit procédé comprenant les étapes consistant à :
a) disposer d'au moins une pluralité de particules polymères absorbantes (APP);
b) disposer d'un support :
c) mettre lesdites particules sur ledit support pour former une macrostructure comprenant une première et une deuxième régions ;
d) disposer d'un ou plusieurs agents de réticulation convenables pour réticuler les particules polymères absorbantes (APP) sur leur surface et/ou entre les particules ;
e) appliquer ledit ou lesdits agents de réticulation auxdites APP dans au moins l'une desdites régions ;
f) ajuster les conditions réactionnelles pour permettre des réactions de réticulation ;
**caractérisé en ce qu'**une ou plusieurs desdites étapes sont exécutées différemment dans ladite première et ladite deuxième régions de façon à créer des régions ayant des propriétés de manipulation de liquide distinctes par création de degrés différents de réticulation en surface et/ou de réticulation entre particules sur ou entre lesdites particules polymères absorbantes (APP) dans lesdites première et deuxième régions.

2. Procédé selon la revendication 1 ou 2, dans lequel lesdites particules polymères absorbantes (APP) de l'étape a) sont différentes dans lesdites première et deuxième régions sur une ou plusieurs des propriétés suivantes :
a1) la composition chimique des particules polymères absorbantes (APP);
a2) la forme physique, la granulométrie moyenne, et/ou la distribution de granulométrie moyenne ;
a) la densité ou la masse surfacique.

3. Procédé selon l'une quelconque des revendications précédentes, dans lequel la densité des particules polymères absorbantes (APP) dans la première région vaut d'environ 0,5 à 2 fois la densité des particules polymères absorbantes (APP) dans la deuxième région.

4. Procédé selon l'une quelconque des revendications précédentes, dans lequel lesdits agents de réticulation tels que mis à disposition dans l'étape d) dans lesdites première et deuxième régions différent sur une ou plusieurs des propriétés suivantes :
d1) la composition chimique de l'agent ;
d2) la concentration de l'agent.

5. Procédé selon l'une quelconque des revendications précédentes, dans lequel ladite application desdits agents de réticulation dans l'étape e) est différente pour ladite première et ladite deuxième régions sur une ou plusieurs des propriétés suivantes :
e1) la quantité d'agent de réticulation appliqué ;
e2) le type d'application.

6. Procédé selon la revendication 5, dans lequel ladite quantité d'agent de réticulation appliqué par gramme de particules polymères absorbantes (APP) dans une région représente au moins le double de la quantité dans l'autre région.

7. Procédé selon l'une quelconque des revendications précédentes, dans lequel ledit ajustement de conditions pour la réaction de réticulation concernant l'étape f) est différent pour lesdites première et deuxième régions sur une ou plusieurs des caractéristiques suivantes :
f1) l'apport d'énergie aux réactifs ;
f2) la durée des conditions réactionnelles ;
f3) la pression sous laquelle se déroule la réaction.

8. Procédé selon la revendication 7, dans lequel ledit apport est sous la forme d'une irradiation électromagnétique, d'électrons émis, ou de chaleur dérivant d'une unité de vulcanisation.

9. Procédé selon la revendication 7 ou 8, dans lequel ledit apport d'énergie déposé dans lesdites régions est différent.

10. Procédé selon la revendication 9, dans lequel la différence d'apport d'énergie est réalisée par utilisation d'un masque qui est placé entre les particules polymères absorbantes (APP) et ladite unité de vulcanisation avec des zones de masquage différentes d'opacité variable pour l'irradiation électromagnétique, les électrons émis, ou la chaleur.
